(19) Europäisches Patentamt
European Patent Office
Office européen des brevets

(11) EP 4 741 503 A1

(12) **EUROPEAN PATENT APPLICATION**
published in accordance with Art. 153(4) EPC

(43) Date of publication:
**13.05.2026 Bulletin 2026/20**

(21) Application number: **24835438.3**

(22) Date of filing: **05.07.2024**

(51) International Patent Classification (IPC):
***C12N 15/113*** *(2010.01)* ***A61K 31/713*** *(2006.01)*
***A61P 9/00*** *(2006.01)*

(52) Cooperative Patent Classification (CPC):
**A61K 31/713; A61P 9/00; C12N 15/113**

(86) International application number:
**PCT/CN2024/103896**

(87) International publication number:
**WO 2025/007960 (09.01.2025 Gazette 2025/02)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **06.07.2023 CN 202310826984**
**19.04.2024 CN 202410478014**

(71) Applicant: **CSPC Zhongqi Pharmaceutical Technology (Shijiazhuang) Co., Ltd.**
**Shijiazhuang, Hebei 050035 (CN)**

(72) Inventors:
- **DONG, Yunxia**
  **Shijiazhuang, Hebei 050035 (CN)**
- **SHAO, Yu**
  **Shijiazhuang, Hebei 050035 (CN)**
- **SU, Xiaoye**
  **Shijiazhuang, Hebei 050035 (CN)**
- **WANG, Lingyu**
  **Shijiazhuang, Hebei 050035 (CN)**
- **DAN, Mo**
  **Shijiazhuang, Hebei 050035 (CN)**
- **ZHANG, Xiaolin**
  **Shijiazhuang, Hebei 050035 (CN)**
- **LIU, Yongmei**
  **Shijiazhuang, Hebei 050035 (CN)**
- **REN, Jiafeng**
  **Shijiazhuang, Hebei 050035 (CN)**
- **WANG, Mingxiao**
  **Shijiazhuang, Hebei 050035 (CN)**

(74) Representative: **Colombo, Stefano Paolo et al**
**Marchi & Partners S.r.l.**
**Via Vittor Pisani, 13**
**20124 Milano (IT)**

Remarks:
The complete document including Reference Table(s) and the Sequence Listing(s) can be downloaded from the EPO website

# (54) RNAI AGENT FOR INHIBITING AGT GENE EXPRESSION AND USE THEREOF

(57) The present invention relates to a double-stranded RNAi agent targeting AGT, a pharmaceutical composition comprising same and a use thereof in preparing a drug for treating diseases associated with AGT expression, such as hypertension.

EP 4 741 503 A1

## Description

### CROSS-REFERENCE TO RELATED APPLICATION

The present application claims the priorities of Chinese Patent Application No. 202310826984.8 entitled with "RNAI AGENT FOR INHIBITING AGT GENE EXPRESSION AND USE THEREOF" and filed with the Chinese Patent Office on July 6, 2023, and Chinese Patent Application No. 202410478014.8 entitled with "RNAI AGENT FOR INHIBITING AGT GENE EXPRESSION AND USE THEREOF" and filed with the Chinese Patent Office on April 19, 2024, the disclosures of which are incorporated herein by reference in their entirety.

### TECHNICAL FIELD

The present application belongs to the field of molecular biology, and relates to a modified double-stranded RNAi agent and use thereof and specifically to a double-stranded RNAi agent for inhibiting the *AGT* gene expression, a pharmaceutical composition thereof, and use of the double-stranded RNAi agent or the pharmaceutical composition thereof for treatment of diseases mediated by *AGT* expression.

### BACKGROUND

RNA interference (RNAi) is ubiquitous across species in nature. Since the initial discovery of the RNAi phenomenon in Caenorhabditis elegans (C. elegans) by Andrew Fire, Craig Mello et al. in 1998 and the confirmation of RNAi in mammals by Tuschl, Phil Sharp *et al.* in 2001, a series of advances have been made in research on the mechanisms and principles, gene functions, clinical applications and the like of RNAi. RNAi plays a pivotal role in multiple organismal protection mechanisms such as defense against viral infection and suppression of transposon jumping (Hutvágner *et al.,* 2001; Elbashir *et al.,* 2001; Zamore 2001). Therefore, products developed based on the RNAi mechanism are highly promising drug candidates. Small interfering RNA (siRNA) could exert an RNA interference effect and is a primary tool for achieving RNAi.

Hypertension, a serious cardiovascular disease, can significantly increase the risk of cardiac, neurological, renal, and other diseases and is a leading cause of premature death worldwide. Hypertension is diagnosed if the systolic blood pressure (SBP) readings are ≥140 mmHg and/or the diastolic blood pressure (DBP) readings are ≥90 mmHg on two separate dates.

It is estimated that 1.28 billion adults aged 30 to 79 years worldwide suffer from hypertension. Of these, 46% of adults with hypertension are unaware of their condition, and only 42% of hypertensive patients have been diagnosed and treated, with only about one-fifth (21%) hypertensive patients have their blood pressure under control. In China, however, the latest disease survey data show that over one-quarter of residents aged 18 or above have hypertension, but the control rate stands at only 16.9%. Prevention and treatment of hypertension are highly challenging. Hypertension could cause damage to major organs such as heart, brain and kidney and is called a "silent killer".

Long-term overactivity of the renin-angiotensin-aldosterone system (RAAS) pathway is considered a major factor in the pathogenesis of cardiovascular diseases, including hypertension and heart failure. While suppression of the RAAS pathway by angiotensin-converting enzyme inhibitors (ACEis) or type I angiotensin receptor blockers (ARBs), such as commonly-used Sartan or Pril antihypertensive drugs, is one of the most effective treatments of hypertension and heart failure with reduced ejection fraction, this approach has limited efficacy in preventing target organ damage and cardiovascular death, and meanwhile carries a higher risk of targeting toxicity, primarily manifested as hyperkalemia and renal insufficiency. Consequently, these drugs can only be taken at lower clinical doses, which restrict their more effective inhibitory effects on these pathways and their clinical benefits. Furthermore, suppression of downstream signaling in the RAAS pathway may induce compensatory activation of upstream pathways, thereby limiting its therapeutic efficacy.

In rodents and humans, angiotensin (1-12) has been identified as a non-renin-dependent substrate for angiotensin II generation. In view of this, a team of researchers from the United States has designed a monoclonal antibody directed against the C-terminus of the human angiotensin (1-12) sequence. The researchers employed a hypertensive model expressing the human angiotensinogen (AGT) gene in the rat genome to investigate the efficacy of immunoneutralization of the endogenous human angiotensin (1-12) in controlling the elevated blood pressure and reversing hypertension-induced target organ damage. They concluded that angiotensin (1-12) is an endogenous substrate for angiotensin II generation and contributes to the regulation of the arterial pressure in the humanized hypertensive model, and that selective immunoneutralization of this alternative substrate is a promising, novel therapeutic approach for treating hypertension and preventing its adverse cardiovascular sequelae.

Targeting the upstream of the RAAS pathway by silencing the hepatic AGT is a novel mechanism for RAAS inhibition. Compared with currently available RAAS inhibitors, this approach boasts two potential advantages. In the first

place, by suppressing the AGT generation in the liver and minimizing the suppression of intrarenal RAAS, and by allowing renal homeostasis and glomerular feedback to remain intact, thereby retaining the potassium ($K^+$) ions and mitigating the renal insufficiency, this approach can exhibit better safety. In the second place, this approach can minimize the escape mechanism that restores the angiotensin II level or maintains the angiotensin II signaling. More complete inhibition of locally generated angiotensin II in the vascular system or cardiac tissue may be conducive to patients with refractory hypertension or heart failure. Therefore, developing a highly efficient AGT-silencing inhibitor with better efficacy, specificity, stability, targeting capability, tolerability, etc. will provide an effective means for subjects with angiotensinogen-associated diseases.

## SUMMARY

An objective of the present application is to provide a double-stranded RNAi agent for inhibiting *AGT* gene expression, a pharmaceutical composition thereof, and methods and uses of the aforementioned double-stranded RNAi agent and pharmaceutical composition thereof for inhibiting or reducing *AGT* gene expression or treating diseases or symptoms mediated by the *AGT* gene.

In one embodiment of the present application, there is provided a double-stranded RNAi agent capable of inhibiting expression of AGT in a cell, wherein the double-stranded RNAi agent comprises a sense strand and an antisense strand, wherein the sense strand is complementary to the antisense strand, and the antisense strand comprises a sequence complementary to a portion of the sequence of an mRNA encoding the AGT, wherein each strand is 14 to 30 nucleotides in length.

For example, each strand (the sense strand or the antisense strand) may be 14 to 30 nucleotides in length, 17 to 30 nucleotides in length, 25 to 30 nucleotides in length, 27 to 30 nucleotides in length, 17 to 23 nucleotides in length, 17 to 21 nucleotides in length, 17 to 19 nucleotides in length, 19 to 25 nucleotides in length, 19 to 23 nucleotides in length, 19 to 21 nucleotides in length, 21 to 25 nucleotides in length or 21 to 23 nucleotides in length.

The duplex region of the double-stranded RNAi agent may be, for example, 14 to 30 nucleotide pairs in length, 17 to 30 nucleotide pairs in length, 27 to 30 nucleotide pairs in length, 17 to 23 nucleotide pairs in length, 17 to 21 nucleotide pairs in length, 17 to 19 nucleotide pairs in length, 19 to 25 nucleotide pairs in length, 19 to 23 nucleotide pairs in length, 19 to 21 nucleotide pairs in length, 21 to 25 nucleotide pairs in length or 21 to 23 nucleotide pairs in length.

In another embodiment, the duplex region has a length selected from 15, 16, 17, 18, 19, 20, 21, 22, 23, 24, 25, 26, and 27 nucleotide pairs.

In one embodiment of the present application, the double-stranded RNAi agent is capable of inhibiting the *AGT* gene expression in humans, monkeys, rats or mice.

The RNAi agent of the present application comprises an RNAi agent having a nucleotide overhang (*i.e.* a reagent having one overhang and one blunt end) at one terminus or having a nucleotide overhang at both termini.

In one embodiment, the double-stranded RNAi agent may contain one or more overhang regions and/or capping groups at the 3'-terminus, 5'-terminus or both termini of one strand or both strands. The overhang may have 1 to 6 nucleotides in length, for example, 2 to 6 nucleotides in length, 1 to 5 nucleotides in length, 2 to 5 nucleotides in length, 1 to 4 nucleotides in length, 2 to 4 nucleotides in length, 1 to 3 nucleotides in length, 2 to 3 nucleotides in length, or 1 to 2 nucleotides in length. The overhang is arbitrarily selected from U, A, G, C, and T.

In one embodiment, the sense strand of the double-stranded RNAi agent has 21 nucleotides, and the antisense strand has 23 nucleotides.

In the present application, the nucleotide sequence of the double-stranded RNAi agent is as set forth in any one of the dsRNA sequences in Table 2.

In one embodiment, the sense strand of the double-stranded RNAi agent is composed of the nucleotide sequence GUCAUCCACAAUGAGAGUACA (SEQ ID NO: 1) (5' to 3' direction) and/or the antisense strand of the double-stranded RNAi agent is composed of the nucleotide sequence UGUACUCUCAUUGUGGAUGACGA (SEQ ID NO: 2) (5' to 3' direction). In one embodiment, the sense strand of the double-stranded RNAi agent is composed of the nucleotide sequence GUCAUCCACAAUGAGAGUACC (SEQ ID NO: 3) (5' to 3' direction) and/or the antisense strand of the double-stranded RNAi agent is composed of the nucleotide sequence GGUACUCUCAUUGUGGAUGACGA (SEQ ID NO: 4) (5' to 3' direction).

In one embodiment, the sense strand of the double-stranded RNAi agent is composed of the nucleotide sequence CUUCUAAUGAGUCGACUUUGA (SEQ ID NO: 5) (5' to 3' direction) and/or the antisense strand of the double-stranded RNAi agent is composed of the nucleotide sequence UCAAAGUCGACUCAUUAGAAGAA (SEQ ID NO: 6) (5' to 3' direction).

In another embodiment, the double-stranded RNAi agent comprises one or more modifications, on one or more nucleotides of the sense strand and the antisense strand thereof, selected from the group consisting of: 2'-methoxyethyl, 2'-O-alkyl, 2'-O-allyl, 2'-C-allyl, 2'-fluoro, 2'-deoxy, 2'-hydroxy, a locked nucleic acid modification, a ring-opened or unlocked nucleic acid modification, a DNA modification, and a fluorescent probe modification.

In one embodiment of the present application, both the sense strand and the antisense strand of the double-stranded RNAi agent contain 2'-O-methyl and/or 2'-fluoro modifications. In another embodiment of the present application, the double-stranded RNAi agent further comprises at least one phosphorothioate or methylphosphonate internucleotide linkage, preferably at least one phosphorothioate linkage.

In another embodiment of the present application, in the double-stranded RNAi agent, the phosphorothioate or methylphosphonate internucleotide linkage is located at the 5'- and 3'-termini of one strand; preferably, the internucleotide linkage is located at the 5'- and 3'-termini of the sense strand and the antisense strand; more preferably, the internucleotide linkage is located within three nucleotides at the 5'- and 3'-termini of the sense strand and the antisense strand.

In another embodiment of the present application, the double-stranded RNAi agent comprises: (1) the antisense strand having an overhang with a 5'(s)mN(s)mN3' structure at the 3'-terminus; (2) the antisense strand bearing a fluoro modification at least at positions 2, 14 and 16 from the 5'-terminus, and a methoxy modification at remaining positions wherever possible; (3) the antisense strand comprising at least two phosphorothioate modifications from the 3'-terminus and 5'-terminus, preferably the antisense strand comprising a phosphorothioate backbone modification in at least the first and second internucleotide linkages from the 3'-terminus and 5'-terminus; (4) the sense strand comprising a fluoro modification at positions 7 and 9 to 11 from the 5'-terminus, and a methoxy modification at remaining positions wherever possible; and (5) the sense strand comprising at least two phosphorothioate modifications from the 5'-terminus, preferably the sense strand comprising a phosphorothioate backbone modification in at least the first and second internucleotide linkages from the 5'-terminus, preferably GalNAc covalently coupled to the 3'-terminus.

In another embodiment of the present application, the double-stranded RNAi agent comprises: (1) a 21-nucleotide sense strand composed of alternating 2'-fluoro-modified regions and 2'-O-methyl-modified regions, each modified region being 1 to 3 nucleotides in length; the first modified regions starting from the 5'-terminus and the 3'-terminus being modified in the same mode; and (2) a 23-nucleotide antisense strand composed of alternating 2'-O-methyl-modified regions and 2'-fluoro-modified regions, each modified region being 1 to 3 nucleotides in length, and all the consecutive nucleotide regions at positions 1 to 3 starting from the 5'-terminus and the 3'-terminus being linked via a phosphorothioate backbone.

In one embodiment of the present application, the double-stranded RNAi agent is conjugated to at least one ligand selected from the group consisting of cholesterol, biotin, vitamins, galactose derivatives or analogs, lactose derivatives or analogs, N-acetylgalactosamine derivatives or analogs, and N-acetylglucosamine (GalNAc) derivatives or analogs.

In some embodiments, the ligand is linked to the 3'-terminus, the 5'-terminus and/or internally within the strand of the double-stranded RNAi agent.

In some embodiments, 1 to 5, 2 to 4, or 3 N-acetylgalactosamine derivatives or analogs thereof are conjugated to the 3'-terminus, the 5'-terminus and/or the internal sites of the double-stranded RNAi agent described above. Specifically, the structure of a single N-acetylgalactosamine derivative is represented by formula I:

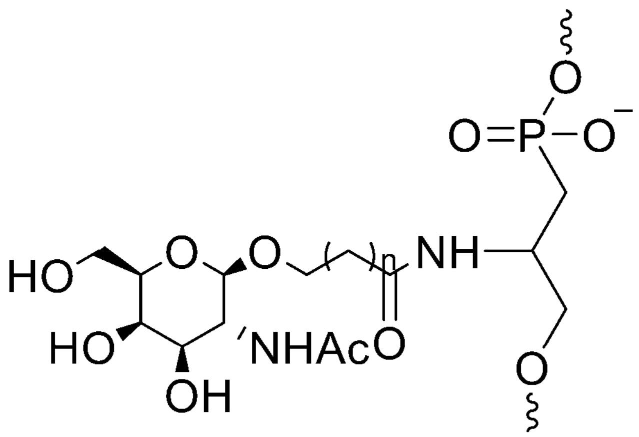

Formula I

wherein n is an integer from 1 to 15.

Preferably, the ligand is linked to the 3'-terminus of the sense strand of the double-stranded RNAi agent.

In one embodiment of the present application, in the double-stranded RNAi agent, the ligand is one or more types of GalNAc derivatives linked to a monovalent or trivalent branched linker.

In one embodiment of the present application, the double-stranded RNAi agent comprises:

(1) an antisense strand composed of a nucleotide sequence having at least 90%, preferably 95%, 96%, 97%, 98%, 99% or 100% identity to the nucleotide sequence UmsGfsUmAmCmUfCmUmCmAmUmUmGmUfGmGfAmUmGmAmCmsGmsAm (SEQ ID NO: 7) and a sense strand composed of a nucleotide sequence having at least 90%, preferably 95%, 96%, 97%, 98%, 99% or 100% identity to the nucleotide sequence GmsUmsCmAmUmCmCfAmCfAfAfUmGmAmGmAmGmUmAmCmAm (SEQ ID NO: 8);

(2) an antisense strand composed of a nucleotide sequence having at least 90%, preferably 95%, 96%, 97%, 98%, 99% or 100% identity to the nucleotide sequence GmsGfsUmAmCmUmCmUmCmAmUmUmGmUfGmGfAmUmGmAmCmsGmsAm (SEQ ID NO: 9) and a sense strand composed of a nucleotide sequence having at least 90%, preferably 95%, 96%, 97%, 98%, 99% or 100% identity to the nucleotide sequence GmsUmsCmAmUmCmCfAmCfAfAfUmGmAmGmAmGmUmAmCmCm (SEQ ID NO: 10);

(3) an antisense strand composed of a nucleotide sequence having at least 90%, preferably 95%, 96%, 97%, 98%, 99% or 100% identity to the nucleotide sequence GmsAfsAmCmCmUmGmUmCmAmAmUmCmUfUmCfUmCmAmGmCmsAmsGm (SEQ ID NO: 11) and a sense strand composed of a nucleotide sequence having at least 90%, preferably 95%, 96%, 97%, 98%, 99% or 100% identity to the nucleotide sequence GmsCmsUmGmAmGmAfAmGfAfUfUmGmAmCmAmGmGmUmUmCm (SEQ ID NO: 12);

(4) an antisense strand composed of a nucleotide sequence having at least 90%, preferably 95%, 96%, 97%, 98%, 99% or 100% identity to the nucleotide sequence UmsGfsAmAmCmCmUmGmUmCmAmAmUmCfUmUfCmUmCmAmGmsCmsAm (SEQ ID NO: 13) and a sense strand composed of a nucleotide sequence having at least 90%, preferably 95%, 96%, 97%, 98%, 99% or 100% identity to the nucleotide sequence CmsUmsGmAmGmAmAfGmAfUfUfGmAmCmAmGmGmUmUmCmAm (SEQ ID NO: 14);

(5) an antisense strand composed of a nucleotide sequence having at least 90%, preferably 95%, 96%, 97%, 98%, 99% or 100% identity to the nucleotide sequence UmsGfsAmUmCmAmUmAmCmAmCmAmGmCfAmAfAmCmAmGmGmsAmsAm (SEQ ID NO: 15) and a sense strand composed of a nucleotide sequence having at least 90%, preferably 95%, 96%, 97%, 98%, 99% or 100% identity to the nucleotide sequence CmsCmsUmGmUmUmUfGmCfUfGfUmGmUmAmUmGmAmUmCmAm (SEQ ID NO: 16);

(6) an antisense strand composed of a nucleotide sequence having at least 90%, preferably 95%, 96%, 97%, 98%, 99% or 100% identity to the nucleotide sequence UmsGfsAmCmAmCmAmUmCmGmCmUmGmAfUmUfUmGmUmCmCmsGmsGm (SEQ ID NO: 17) and a sense strand composed of a nucleotide sequence having at least 90%, preferably 95%, 96%, 97%, 98%, 99% or 100% identity to the nucleotide sequence GmsGmsAmCmAmAmAfUmCfAfGfCmGmAmUmGmUmGmUmCmAm (SEQ ID NO: 18);

(7) an antisense strand composed of a nucleotide sequence having at least 90%, preferably 95%, 96%, 97%, 98%, 99% or 100% identity to the nucleotide sequence AmsGfsAmAmGmAmAmAmAmGmGmGmUmGmGfGmAfGmAmCmUmGmsGmsGm (SEQ ID NO: 19) and a sense strand composed of a nucleotide sequence having at least 90%, preferably 95%, 96%, 97%, 98%, 99% or 100% identity to the nucleotide sequence CmsAmsGmUmCmUmCfCmCfAfCfCmUmUmUmUmCmUmUmCmUm (SEQ ID NO: 20);

(8) an antisense strand composed of a nucleotide sequence having at least 90%, preferably 95%, 96%, 97%, 98%, 99% or 100% identity to the nucleotide sequence CmsAfsUmUmAmGmAmAmGmAmAmAmAmGfGmUfGmGmGmAmGmsAmsCm (SEQ ID NO: 21) and a sense strand composed of a nucleotide sequence having at least 90%, preferably 95%, 96%, 97%, 98%, 99% or 100% identity to the nucleotide sequence CmsUmsCmCmCmAmCfCmUfUfUfUmCmUmUmCmUmAmAmUmGm (SEQ ID NO: 22);

(9) an antisense strand composed of a nucleotide sequence having at least 90%, preferably 95%, 96%, 97%, 98%, 99% or 100% identity to the nucleotide sequence UmsCfsAmAmAmGmUmCmGmAmCmUmCmAfUmUfAmGmAmAmGmsAmsAm (SEQ ID NO: 23) and a sense strand composed of a nucleotide sequence having at least 90%, preferably 95%, 96%, 97%, 98%, 99% or 100% identity to the nucleotide sequence CmsUmsUmCmUmAmAfUmGfAfGfUmCmGmAmCmUmUmUmGmAm (SEQ ID NO: 24);

(10) an antisense strand composed of a nucleotide sequence having at least 90%, preferably 95%, 96%, 97%, 98%, 99% or 100% identity to the nucleotide sequence AmsCfsAmCmUmUmAmGmAmCmCmAmAmGfGmAfGmAmAmAmCmsGmsGm (SEQ ID NO: 25) and a sense strand composed of a nucleotide sequence having at least 90%, preferably 95%, 96%, 97%, 98%, 99% or 100% identity to the nucleotide sequence GmsUmsUmCmUmCfCmUfUfGfGmUmCmUmAmAmGmUmGmUm (SEQ ID NO: 26); or

(11) an antisense strand composed of a nucleotide sequence having at least 90%, preferably 95%, 96%, 97%, 98%, 99% or 100% identity to the nucleotide sequence UmsUfsUmUmUmUmGmUmUmUmCmAmCmAfAmAfCmAmAmGmCmsUmsGm (SEQ ID NO: 27) and a sense strand composed of a nucleotide sequence having at least 90%, preferably 95%, 96%, 97%, 98%, 99% or 100% identity to the nucleotide sequence GmsCmsUmUmGmUmUfUmGfUfGfAmAmAmCmAmAmAmAmAmAm (SEQ ID NO: 28);

wherein Am, Um, Cm and Gm represent 2'-O-methyl-modified ribonucleotides A, U, C and G, respectively; Af, Uf, Cf and Gf represent 2'-fluoro-modified ribonucleotides A, U, C and G, respectively; and (s) represents that two adjacent

nucleotides are linked via a phosphorothioate backbone.

Furthermore, the present application provides a double-stranded RNAi agent comprising a sense strand and an antisense strand, wherein:

(1) the sense strand comprises

UmsGfsUmAmCmUfCmUmCmAmUmUmGmUfGmGfAmUmGmAmCmsGmsAm (SEQ ID NO: 7), and the antisense strand comprises GmsUmsCmAmUmCmCfAmCfAfAfUmGmAmGmAmGmUmAmCmAm (SEQ ID NO: 8);

(2) the sense strand comprises

GmsGfsUmAmCmUmCmUmCmAmUmUmGmUfGmGfAmUmGmAmCmsGmsAm (SEQ ID NO: 9), and the antisense strand comprises GmsUmsCmAmUmCmCfAmCfAfAfUmGmAmGmAmGmUmAmCmCm (SEQ ID NO: 10);

(3) the sense strand comprises

GmsAfsAmCmCmUmGmUmCmAmAmUmCmUfUmCfUmCmAmGmCmsAmsGm (SEQ ID NO: 11), and the antisense strand comprises GmsCmsUmGmAmGmAfAmGfAfUfUmGmAmCmAmGmGmUmUmCm (SEQ ID NO: 12);

(4) the sense strand comprises

UmsGfsAmAmCmCmUmGmUmCmAmAmUmCfUmUfCmUmCmAmGmsCmsAm (SEQ ID NO: 13), and the antisense strand comprises CmsUmsGmAmGmAmAfGmAfUfUfGmAmCmAmGmGmUmUmCmAm (SEQ ID NO: 14);

(5) the sense strand comprises

UmsGfsAmUmCmAmUmAmCmAmCmAmGmCfAmAfAmCmAmGmGmsAmsAm (SEQ ID NO: 15), and the antisense strand comprises CmsCmsUmGmUmUmUfGmCfUfGfUmGmUmAmUmGmAmUmCmAm (SEQ ID NO: 16);

(6) the sense strand comprises

UmsGfsAmCmAmCmAmUmCmGmCmUmGmAfUmUfUmGmUmCmCmsGmsGm (SEQ ID NO: 17), and the antisense strand comprises GmsGmsAmCmAmAmAfUmCfAfGfCmGmAmUmGmUmGmUmCmAm (SEQ ID NO: 18);

(7) the sense strand comprises

AmsGfsAmAmGmAmAmAmAmGmGmUmGmGfGmAfGmAmCmUmGmsGmsGm (SEQ ID NO: 19), and the antisense strand comprises CmsAmsGmUmCmUmCfCmCfAfCfCmUmUmUmUmCmUmUmCmUm (SEQ ID NO: 20);

(8) the sense strand comprises

CmsAfsUmUmAmGmAmAmGmAmAmAmAmGfGmUfGmGmGmAmGmsAmsCm (SEQ ID NO: 21), and the antisense strand comprises CmsUmsCmCmCmAmCfCmUfUfUfUmCmUmUmCmUmAmAmUmGm (SEQ ID NO: 22);

(9) the sense strand comprises

UmsCfsAmAmAmGmUmCmGmAmCmUmCmAfUmUfAmGmAmAmGmsAmsAm (SEQ ID NO: 23), and the antisense strand comprises CmsUmsUmCmUmAmAfUmGfAfGfUmCmGmAmCmUmUmUmGmAm (SEQ ID NO: 24);

(10) the sense strand comprises

AmsCfsAmCmUmUmAmGmAmCmCmAmAmGfGmAfGmAmAmAmCmsGmsGm (SEQ ID NO: 25), and the antisense strand comprises GmsUmsUmUmCmUmCfCmUfUfGfGmUmCmUmAmAmGmUmGmUm (SEQ ID NO: 26); or

(11) the sense strand comprises

UmsUfsUmUmUmUmGmUmUmUmCmAmCmAfAmAfCmAmAmGmCmsUmsGm (SEQ ID NO: 27), and the antisense strand comprises GmsCmsUmUmGmUmUfUmGfUfGfAmAmAmCmAmAmAmAmAmAm (SEQ ID NO: 28);

wherein Am, Um, Cm and Gm represent 2'-O-methyl-modified ribonucleotides A, U, C and G, respectively; Af, Uf, Cf and Gf represent 2'-fluoro-modified ribonucleotides A, U, C and G, respectively; and (s) represents that two adjacent nucleotides are linked via a phosphorothioate backbone.

In some embodiments, the double-stranded RNAi agent comprises a sense strand and an antisense strand, wherein:

(1) the nucleic acid sequence of the sense strand is composed of UmsGfsUmAmCmUfCmUmCmAmUmUmGmUfGmGfAmUmGmAmCmsGmsAm (SEQ ID NO: 7) and 0, 1, 2, 3 or 4 nucleotides, and the nucleotide sequence of the antisense strand is composed of GmsUmsCmAmUmCmCfAmCfAfAfUmGmAmGmAmGmUmAmCmAm (SEQ ID NO: 8) and 0, 1, 2, 3 or 4 nucleotides;
(2) the nucleic acid sequence of the sense strand is composed of GmsGfsUmAmCmUmCmUmCmAmUmUmGmUfGmGfAmUmGmAmCmsGmsAm (SEQ ID NO: 9) and 0, 1, 2, 3 or 4 nucleotides, and the nucleic acid sequence of the antisense strand is composed of GmsUmsCmAmUmCmCfAmCfAfAfUmGmAmGmAmGmUmAmCmCm (SEQ ID NO: 10) and 0, 1, 2, 3 or 4 nucleotides;
(3) the nucleic acid sequence of the sense strand is composed of GmsAfsAmCmCmUmGmUmCmAmAmUmCmUfUmCfUmCmAmGmCmsAmsGm (SEQ ID NO: 11) and 0, 1, 2, 3 or 4 nucleotides, and the nucleic acid sequence of the antisense strand is composed of GmsCmsUmGmAmGmAfAmGfAfUfUmGmAmCmAmGmGmUmUmCm (SEQ ID NO: 12) and 0, 1, 2, 3 or 4 nucleotides;
(4) the nucleic acid sequence of the sense strand is composed of UmsGfsAmAmCmCmUmGmUmCmAmAmUmCfUmUfCmUmCmAmGmsCmsAm (SEQ ID NO: 13) and 0, 1, 2, 3 or 4 nucleotides, and the nucleic acid sequence of the antisense strand is composed of CmsUmsGmAmGmAmAfGmAfUfUfGmAmCmAmGmGmUmUmCmAm (SEQ ID NO: 14) and 0, 1, 2, 3 or 4 nucleotides;
(5) the nucleic acid sequence of the sense strand is composed of UmsGfsAmUmCmAmUmAmCmAmCmAmGmCfAmAfAmCmAmGmGmsAmsAm (SEQ ID NO: 15) and 0, 1, 2, 3 or 4 nucleotides, and the nucleic acid sequence of the antisense strand is composed of CmsCmsUmGmUmUmUfGmCfUfGfUmGmUmAmUmGmAmUmCmAm (SEQ ID NO: 16) and 0, 1, 2, 3 or 4 nucleotides;
(6) the nucleic acid sequence of the sense strand is composed of UmsGfsAmCmAmCmAmUmCmGmCmUmGmAfUmUfUmGmUmCmCmsGmsGm (SEQ ID NO: 17) and 0, 1, 2, 3 or 4 nucleotides, and the nucleic acid sequence of the antisense strand is composed of GmsGmsAmCmAmAmAfUmCfAfGfCmGmAmUmGmUmGmUmCmAm (SEQ ID NO: 18) and 0, 1, 2, 3 or 4 nucleotides;
(7) the nucleic acid sequence of the sense strand is composed of AmsGfsAmAmGmAmAmAmAmGmGmUmGmGfGmAfGmAmCmUmGmsGmsGm (SEQ ID NO: 19) and 0, 1, 2, 3 or 4 nucleotides, and the nucleic acid sequence of the antisense strand is composed of CmsAmsGmUmCmUmCfCmCfAfCfCmUmUmUmUmCmUmUmCmUm (SEQ ID NO: 20) and 0, 1, 2, 3 or 4 nucleotides;
(8) the nucleic acid sequence of the sense strand is composed of CmsAfsUmUmAmGmAmAmGmAmAmAmAmGfGmUfGmGmGmAmGmsAmsCm (SEQ ID NO: 21) and 0, 1, 2, 3 or 4 nucleotides, and the nucleic acid sequence of the antisense strand is composed of CmsUmsCmCmCmAmCfCmUfUfUfUmCmUmUmCmUmAmAmUmGm (SEQ ID NO: 22) and 0, 1, 2, 3 or 4 nucleotides;
(9) the nucleic acid sequence of the sense strand is composed of UmsCfsAmAmAmGmUmCmGmAmCmUmCmAfUmUfAmGmAmAmGmsAmsAm (SEQ ID NO: 23) and 0, 1, 2, 3 or 4 nucleotides, and the nucleic acid sequence of the antisense strand is composed of CmsUmsUmCmUmAmAfUmGfAfGfUmCmGmAmCmUmUmUmGmAm (SEQ ID NO: 24) and 0, 1, 2, 3 or 4 nucleotides;
(10) the nucleic acid sequence of the sense strand is composed of AmsCfsAmCmUmUmAmGmAmCmCmAmAmGfGmAfGmAmAmAmCmsGmsGm (SEQ ID NO: 25) and 0, 1, 2, 3 or 4 nucleotides, and the nucleic acid sequence of the antisense strand is composed of GmsUmsUmUmCmUmCfCmUfUfGfGmUmCmUmAmAmGmUmGmUm (SEQ ID NO: 26) and 0, 1, 2, 3 or 4 nucleotides; or

(11) the nucleic acid sequence of the sense strand is composed of UmsUfsUmUmUmUmGmUmUmUmCmAmCmAfAmAfCmAmAmGmCmsUmsGm (SEQ ID NO: 27) and 0, 1, 2, 3 or 4 nucleotides, and the nucleic acid sequence of the antisense strand is composed of GmsCmsUmUmGmUmUfUmGfUfGfAmAmAmCmAmAmAmAmAmAm (SEQ ID NO: 28) and 0, 1, 2, 3 or 4 nucleotides;

wherein Am, Um, Cm and Gm represent 2'-O-methyl-modified ribonucleotides A, U, C and G, respectively; Af, Uf, Cf and Gf represent 2'-fluoro-modified ribonucleotides A, U, C and G, respectively; and (s) represents that two adjacent nucleotides are linked via a phosphorothioate backbone.

Furthermore, in some embodiments, 1 to 5, 2 to 4, or 3 N-acetylgalactosamine derivatives or analogs are attached to the 3'-terminus of the sense strand of the double-stranded RNAi agent. Specifically, the structure of a single N-acetylgalactosamine derivative is represented by formula I:

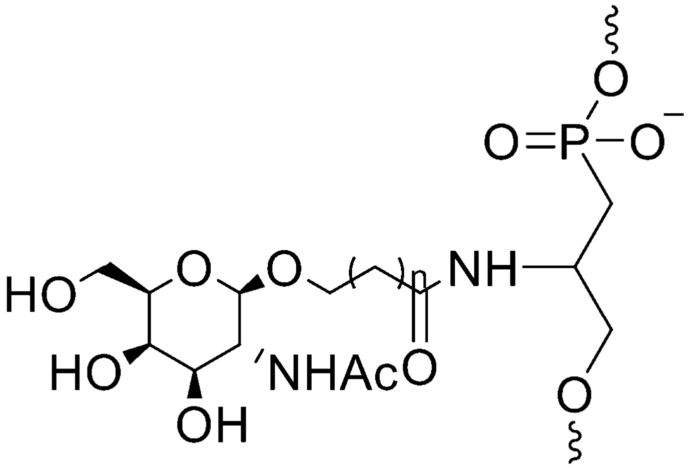

Formula I

wherein, n is an integer from 1 to 15.

In some embodiments of the present application, in the double-stranded RNAi agent, 1 to 5, 2 to 4, or 3 GalNAc derivatives of one or more types,linked to a monovalent or trivalent branched linker, are attached to the 3'-terminus of the sense strand of the double-stranded RNAi agent.

In some embodiments of the present application, in the double-stranded RNAi agent, the GalNAc structure is represented by formula II:

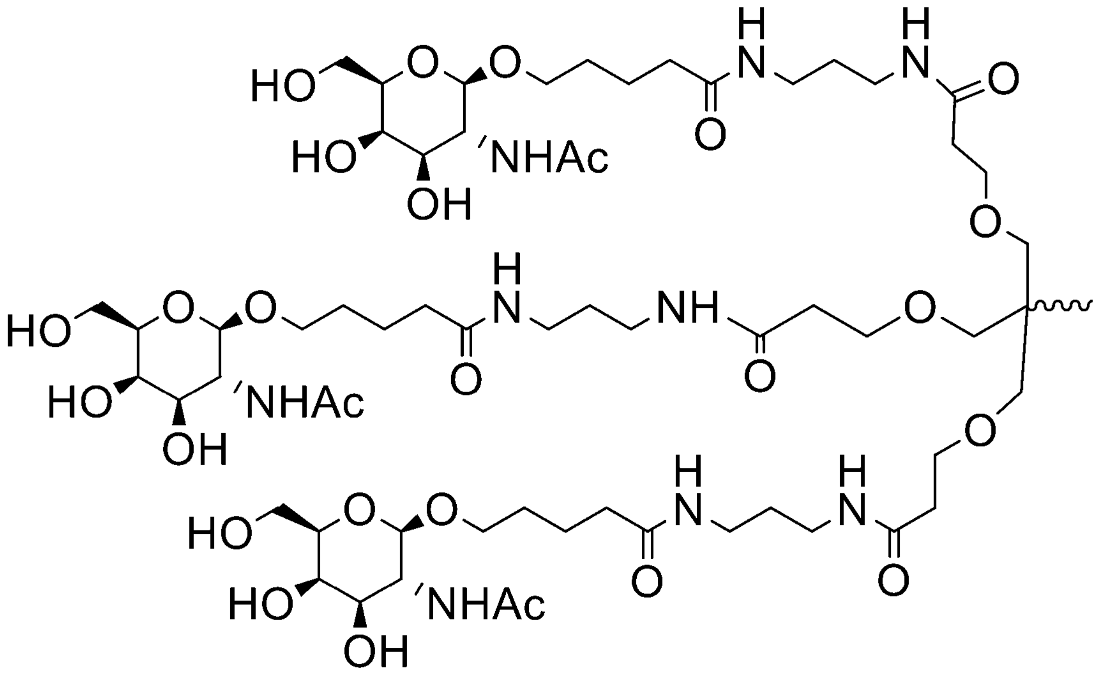

Formula II

In some embodiments of the present application, the nucleotide at the 3'-terminus of the sense strand of the aforementioned double-stranded RNAi agent is linked to L96 having the structural formula represented by formula III:

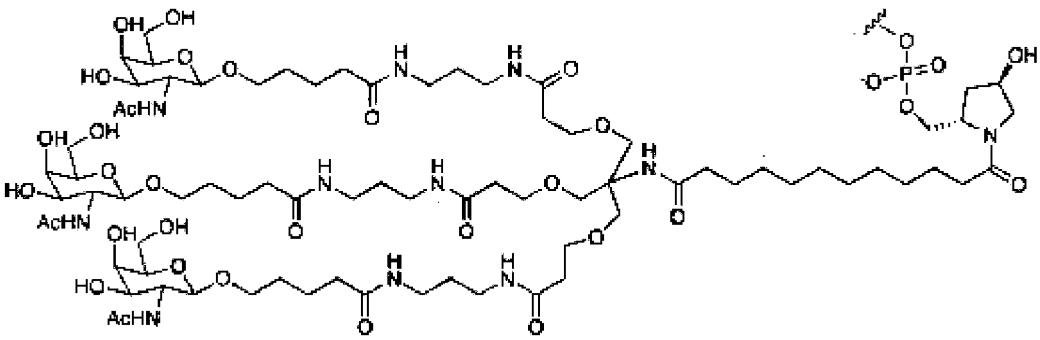

Formula III

In one embodiment of the present application, the double-stranded RNAi agent comprises:

(1) an antisense strand composed of a nucleotide sequence having at least 90%, preferably 95%, 96%, 97%, 98%, 99% or 100% identity to the nucleotide sequence UmsGfsUmAmCmUfCmUmCmAmUmUmGmUfGmGfAmUmGmAmCmsGmsAm (SEQ ID NO: 7) and a sense strand composed of a nucleotide sequence having at least 90%, preferably 95%, 96%, 97%, 98%, 99% or 100% identity to the nucleotide sequence GmsUmsCmAmUmCmCfAmCfAfAfUmGmAmGmAmGmUmAmCmAm-L96 (SEQ ID NO: 29);
(2) an antisense strand composed of a nucleotide sequence having at least 90%, preferably 95%, 96%, 97%, 98%, 99% or 100% identity to the nucleotide sequence GmsGfsUmAmCmUmCmUmCmAmUmUmGmUfGmGfAmUmGmAmCmsGmsAm (SEQ ID NO: 9) and a sense strand composed of a nucleotide sequence having at least 90%, preferably 95%, 96%, 97%, 98%, 99% or 100% identity to the nucleotide sequence GmsUmsCmAmUmCmCfAmCfAfAfUmGmAmGmAmGmUmAmCmCm-L96 (SEQ ID NO: 30);
(3) an antisense strand composed of a nucleotide sequence having at least 90%, preferably 95%, 96%, 97%, 98%, 99% or 100% identity to the nucleotide sequence GmsAfsAmCmCmUmGmUmCmAmAmUmCmUfUmCfUmCmAmGmCmsAmsGm (SEQ ID NO: 11) and a sense strand composed of a nucleotide sequence having at least 90%, preferably 95%, 96%, 97%, 98%, 99% or 100% identity to the nucleotide sequence GmsCmsUmGmAmGmAfAmGfAfUfUmGmAmCmAmGmGmUmUmCm-L96 (SEQ ID NO: 31);

(4) an antisense strand composed of a nucleotide sequence having at least 90%, preferably 95%, 96%, 97%, 98%, 99% or 100% identity to the nucleotide sequence UmsGfsAmAmCmCmUmGmUmCmAmAmUmCfUmUfCmUmC mAmGmsCmsAm (SEQ ID NO: 13) and a sense strand composed of a nucleotide sequence having at least 90%, preferably 95%, 96%, 97%, 98%, 99% or 100% identity to the nucleotide sequence CmsUmsGmAmGmAmAfGmAfUfUfGmAmCmAmGmGmUmUmCmAm-L96 (SEQ ID NO: 32);

(5) an antisense strand composed of a nucleotide sequence having at least 90%, preferably 95%, 96%, 97%, 98%, 99% or 100% identity to the nucleotide sequence UmsGfsAmUmCmAmUmAmCmAmCmAmGmCfAmAfAmCmA mGmGmsAmsAm (SEQ ID NO: 15) and a sense strand composed of a nucleotide sequence having at least 90%, preferably 95%, 96%, 97%, 98%, 99% or 100% identity to the nucleotide sequence CmsCmsUmGmUmUmUfGmCfUfGfUmGmUmAmUmGmAmUmCmAm-L96 (SEQ ID NO: 33);

(6) an antisense strand composed of a nucleotide sequence having at least 90%, preferably 95%, 96%, 97%, 98%, 99% or 100% identity to the nucleotide sequence UmsGfsAmCmAmCmAmUmCmGmCmUmGmAfUmUfUmGmU mCmCmsGmsGm (SEQ ID NO: 17) and a sense strand composed of a nucleotide sequence having at least 90%, preferably 95%, 96%, 97%, 98%, 99% or 100% identity to the nucleotide sequence GmsGmsAmCmAmAmAfUmCfAfGfCmGmAmUmGmUmGmUmCmAm-L96 (SEQ ID NO: 34);

(7) an antisense strand composed of a nucleotide sequence having at least 90%, preferably 95%, 96%, 97%, 98%, 99% or 100% identity to the nucleotide sequence AmsGfsAmAmGmAmAmAmAmGmGmUmGmGfGmAfGmAmC mUmGmsGmsGm (SEQ ID NO: 19) and a sense strand composed of a nucleotide sequence having at least 90%, preferably 95%, 96%, 97%, 98%, 99% or 100% identity to the nucleotide sequence CmsAmsGmUmCmUmCfCmCfAfCfCmUmUmUmUmCmUmUmCmUm-L96 (SEQ ID NO: 35);

(8) an antisense strand composed of a nucleotide sequence having at least 90%, preferably 95%, 96%, 97%, 98%, 99% or 100% identity to the nucleotide sequence CmsAfsUmUmAmGmAmAmGmAmAmAmAmGfGmUfGmGmG mAmGmsAmsCm (SEQ ID NO: 21) and a sense strand composed of a nucleotide sequence having at least 90%, preferably 95%, 96%, 97%, 98%, 99% or 100% identity to the nucleotide sequence CmsUmsCmCmCmAmCfCmUfUfUfUmCmUmUmCmUmAmAmUmGm-L96 (SEQ ID NO: 36);

(9) an antisense strand composed of a nucleotide sequence having at least 90%, preferably 95%, 96%, 97%, 98%, 99% or 100% identity to the nucleotide sequence UmsCfsAmAmAmGmUmCmGmAmCmUmCmAfUmUfAmGmA mAmGmsAmsAm (SEQ ID NO: 23) and a sense strand composed of a nucleotide sequence having at least 90%, preferably 95%, 96%, 97%, 98%, 99% or 100% identity to the nucleotide sequence CmsUmsUmCmUmAmAfUmGfAfGfUmCmGmAmCmUmUmUmGmAm-L96 (SEQ ID NO: 37);

(10) an antisense strand composed of a nucleotide sequence having at least 90%, preferably 95%, 96%, 97%, 98%, 99% or 100% identity to the nucleotide sequence AmsCfsAmCmUmUmAmGmAmCmCmAmAmGfGmAfGmAmA mAmCmsGmsGm (SEQ ID NO: 25) and a sense strand composed of a nucleotide sequence having at least 90%, preferably 95%, 96%, 97%, 98%, 99% or 100% identity to the nucleotide sequence GmsUmsUmUmCmUmCfCmUfUfGfGmUmCmUmAmAmGmUmGmUm-L96 (SEQ ID NO: 38); or

(11) an antisense strand composed of a nucleotide sequence having at least 90%, preferably 95%, 96%, 97%, 98%, 99% or 100% identity to the nucleotide sequence UmsUfsUmUmUmUmGmUmUmUmCmAmCmAfAmAfCmAmA mGmCmsUmsGm (SEQ ID NO: 27) and a sense strand composed of a nucleotide sequence having at least 90%, preferably 95%, 96%, 97%, 98%, 99% or 100% identity to the nucleotide sequence GmsCmsUmUmGmUmUfUmGfUfGfAmAmAmCmAmAmAmAmAmAm-L96 (SEQ ID NO: 39);

wherein Am, Um, Cm and Gm represent 2'-O-methyl-modified ribonucleotides A, U, C and G, respectively; Af, Uf, Cf and Gf represent 2'-fluoro-modified ribonucleotides A, U, C and G, respectively; and (s) represents that two adjacent nucleotides are linked via a phosphorothioate backbone, and L96 is linked to the 3'-terminus of the sense strand of the double-stranded RNAi agent and has the structure represented by formula III:

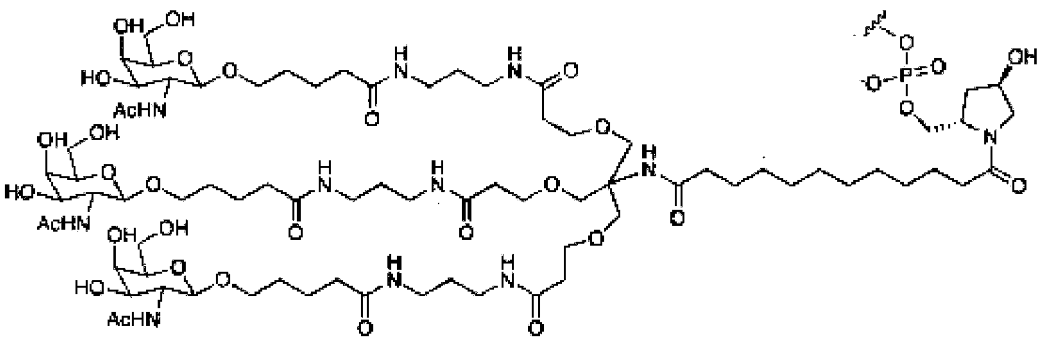

Formula III

In one embodiment of the present application, the aforementioned double-stranded RNAi agent has a structure of formula IV:

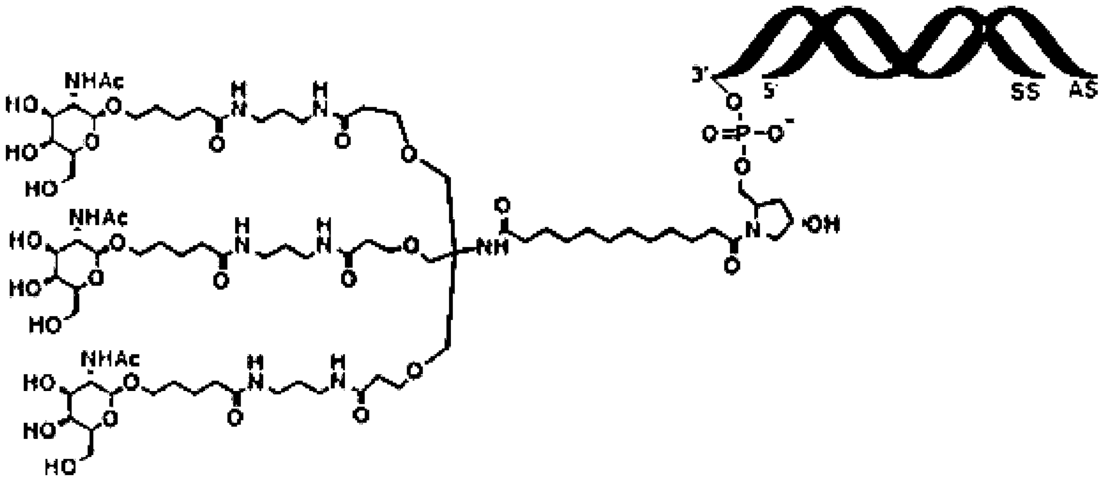

Formula IV

The present application provides the following examples of the double-stranded RNAi agent: AGT203-E-G, AGT202-E-G, AGT814-E-G, AGT815-E-G, AGT1452-E-G, AGT1613-EG, AGT1638-E-G, AGT1642-E-G, AGT1654-E-G, AGT1689-E-G, and AGT1854-E-G.

The present application further comprises a DNA molecule capable of generating the aforementioned double-stranded RNAi agent, a vector capable of expressing the double-stranded RNAi agent, and a reagent or kit containing the double-stranded RNAi agent or the DNA molecule or the vector.

The present application further provides a cell containing the aforementioned double-stranded RNAi agent.

The present application additionally provides a pharmaceutical composition comprising the aforementioned double-stranded RNAi agent.

The pharmaceutical composition comprises a pharmacologically effective amount of the double-stranded RNAi agent according to the present application and other pharmaceutically acceptable components. The "effective amount" refers to an amount of the double-stranded RNAi agent effective to produce a desired pharmacological therapeutic effect. "Other components" include water, saline, dextrose, buffer solutions (*e.g.* PBS), excipients, diluents, disintegrants, binders, lubricants, sweeteners, flavoring agents, preservatives, or combinations thereof.

The present application further provides a method for inhibiting *AGT* expression in a cell, the method comprising: (a) contacting the cell with the aforementioned double-stranded RNAi agent or pharmaceutical composition thereof; (b) maintaining the cell generated in step (a) for a period of time sufficient to allow for degradation of an mRNA transcript of the *AGT* gene, thereby inhibiting the expression of the *AGT* gene in the cell.

The present application provides use of the aforementioned double-stranded RNAi agent or pharmaceutical composition thereof in inhibition of *AGT* gene expression or preparation of a product for inhibiting *AGT* gene expression, wherein the inhibition of the *AGT* gene expression is inhibition or reduction of the expression level of the *AGT* gene of the human, monkey, rat or mouse in the *in vivo* or *in vitro* cell. The cell is an AGT-expressing mammalian cell, such as primate cells or human cells. Preferably, the *AGT* gene is expressed at a high level in the target cell. More preferably, the cell is derived from the brain, salivary glands, heart, spleen, lungs, liver, kidneys, intestines or tumors. Yet more preferably, the cell is a liver cancer cell or cervical cancer cell. Still more preferably, the cell is selected from HepG2, Hep3B, Huh7, COS7, 293T, MHCC97H, Hela, mouse primary hepatocytes, and human primary hepatocytes. In some embodiments of the present application, the final cell concentration of the double-stranded RNAi agent is 0.1 to 1000 nM, *e.g.* 10 to 500 nM, 25 to 300 nM, or 50 to 100 nM.

In some embodiments of the present application, the double-stranded RNAi agent and the pharmaceutical composition thereof can be administered by any suitable means, such as parenteral administration including intramuscular, intravenous, arterial, peritoneal or subcutaneous injection. The mode of administration includes, but is not limited to, a single dose or multiple doses.

The present application further provides use of the aforementioned double-stranded RNAi agent or the pharmaceutical composition thereof in preparation of a medicament for preventing and/or treating a disease mediated by *AGT* expression or alleviating symptoms of a disease mediated by the *AGT* gene.

The present application further provides the aforementioned double-stranded RNAi agent or the pharmaceutical composition thereof for use in preventing and/or treating a disease mediated by *AGT* expression or for use in alleviating

symptoms of a disease mediated by the *AGT* gene.

The present application also provides a method for preventing and/or treating a disease mediated by *AGT* expression or a method for alleviating symptoms of a disease mediated by the *AGT* gene, comprising administering, to a subject in need thereof, the double-stranded RNAi agent according to the present application or a pharmaceutical composition thereof.

In the present application, the disease mediated by the *AGT* gene includes hypertension, hypertension, borderline hypertension, primary hypertension, secondary hypertension, hypertensive emergency, hypertensive urgency, isolated systolic or diastolic hypertension, pregnancy-associated hypertension, diabetic hypertension, resistant hypertension, refractory hypertension, paroxysmal hypertension, renovascular hypertension, Goldblatt hypertension, ocular hypertension, glaucoma, pulmonary hypertension, portal hypertension, systemic venous hypertension, systolic hypertension, labile hypertension; hypertensive heart disease, hypertensive nephropathy, atherosclerosis, arteriosclerosis, vasculopathy, diabetic nephropathy, diabetic retinopathy, chronic heart failure, cardiomyopathy, diabetic cardiac myopathy, glomerulosclerosis, coarctation of the aorta, aortic aneurism, ventricular fibrosis, Cushing's syndrome, and other glucocorticoid excess states including chronic steroid therapy, pheochromocytoma, reninoma, secondary aldosteronism and other mineralocorticoid excess states, sleep apnea, thyroid/parathyroid disease, heart failure, myocardial infarction, angina, stroke, diabetes mellitus, renal disease, renal failure, systemic sclerosis, intrauterine growth restriction (IUGR), and fetal growth restriction.

In some embodiments, a single dose of the pharmaceutical composition may be sustained over a long period of time, with the reduction in the *AGT* expression level sustained for at least 4, 7, 11, 14, 18, 21, 25, 28, 32, 35, 39, 42, 49, 56 days or longer.

The present application possesses the following innovations: 1. RNAi molecules obtained by high-throughput screening exhibit an inhibitory activity comparable to or higher than that of AD85481 (a positive control compound). 2. Modified RNAi molecules have high stability and a high inhibitory activity. 3. The pharmacodynamic experiment on rhesus monkeys has proved that compared with the positive control AD85481-E-G, the candidate sequences can reduce the serum AGT protein level by 90% or more, and exhibit a longer persistent period and a significant effect.

It is to be noted that while numerous modifications could be attempted to improve the performance of the double-stranded RNAi, these attempts rarely yield a double-stranded RNAi that not only mediates RNA interference but also has improved stability (*e.g.* increased resistance to nucleases and/or a prolonged persistent period) in serum. The modified double-stranded RNAi of the present application not only has high stability but also maintains a high inhibitory activity.

### Definitions

"AGT" refers to an angiotensinogen gene or protein. AGT is also referred to as ANHU, hFLT1 or SERPINA8. Examples of the AGT mRNA sequence are readily available, *e.g.,* from GenBank.

"G", "C", "A", and "U" typically represent nucleotides containing guanine, cytosine, adenine, and uracil as bases, respectively. However, it should be appreciated that the term "ribonucleotide" or "nucleotide" or "deoxyribonucleotide" may also refer to a modified nucleotide or an alternative replacement moiety. Those skilled in the art should be well aware that guanine, cytosine, adenine, and uracil may be replaced by other moieties without substantially altering the base-pairing properties of an oligonucleotide (including a nucleotide having such a replacement moiety). Sequences containing such replacement moieties are embodiments of the present application.

"RNAi agent" refers to an agent that mediate the targeted cleavage of an RNA transcript via an RNA-induced silencing complex (RISC) pathway. The RNAi agent guides the sequence-specific degradation of mRNA through a process known as RNA interference (RNAi). The RNAi agent regulates (*e.g.* inhibits) the expression of *AGT* in cells, such as cells in a subject (*e.g.* a mammalian subject).

In one embodiment, the RNAi agent of the present application includes a single-stranded RNA that interacts with a target RNA sequence (such as the target mRNA sequence of AGT) to guide cleavage of the target RNA.

In another embodiment, the RNAi agent may be a single-stranded siRNA introduced into a cell or organism to inhibit the target mRNA.

In another embodiment, the "RNAi agents" used in the composition, use, and method of the present application are double-stranded RNAs, and refer to "double-stranded RNAi agents" herein. The term "double-stranded RNAi agent" refers to a complex of ribonucleic acid molecules, which has a double-stranded structure comprising two antiparallel and substantially complementary nucleic acid strands, and is referred to as having "sense" and "antisense" orientations relative to the target RNA, *i.e.* the *AGT* gene. In some embodiments of the present application, the double-stranded RNA triggers degradation of a target RNA, such as mRNA, through a post-transcriptional gene silencing mechanism (referred to as RNA interference or RNAi herein).

"Antisense strand" refers to a strand of a double-stranded RNAi agent that comprises a region substantially complementary to a target sequence (such as a human AGT mRNA).

"Sense strand" refers to a dsRNA strand containing a region that is substantially complementary to the region of

the antisense strand.

"Complementary region" refers to a region on the antisense strand that is completely or substantially complementary to the target mRNA sequence. Where the complementary region is not perfectly complementary to the target sequence, mismatches may occur in the internal or terminal regions of the molecule. As used herein, the term "complementary" refers to the ability of a first polynucleotide to hybridize with a second polynucleotide under certain conditions, such as stringency conditions.

A "nucleotide overhang" refers to one or more unpaired nucleotides that protrude from the duplex structure of an RNAi agent when the 3'-terminus of one strand of the RNAi agent extends beyond the 5'-terminus of the other strand, or vice versa. "Blunt" or "blunt end" means that there are no unpaired nucleotides (*i.e.* no nucleotide overhangs) at that end of the double-stranded RNAi agent. A "blunt-ended" RNAi agent is a dsRNA that is double-stranded over its extire length, that is, there is no nucleotide overhang at either end of this molecule.

"Inhibition of *AGT* expression in cells" includes inhibition of the expression of any *AGT* gene (*e.g.* a mouse *AGT* gene, a rat *AGT* gene, a monkey *AGT* gene, or a human *AGT* gene) and variants *(e.g.* naturally occurring variants) or mutants of the *AGT* gene. Therefore, this *AGT gene* may be a wild-type *AGT* gene, a mutant *AGT* gene, or a transgenic *AGT* gene in the case of a genetically manipulated cell, population of cells, or organism.

The cell is an *AGT*-expressing mammalian cell, such as primate cells or human cells. Preferably, the *AGT* gene is expressed at a high level in the target cell. More preferably, the cell is derived from the brain, salivary glands, heart, spleen, lungs, liver, kidneys, intestines or tumors. Yet more preferably, the cell is a liver cancer cell or cervical cancer cell. Still more preferably, the cell is selected from HepG2, Hep3B, Huh7, COS7, 293T, MHCC97H, Hela, mouse primary hepatocytes, and human primary hepatocytes.

"Inhibition of the *AGT* gene expression" includes inhibition of the *AGT* gene at any level, for example, at least partial inhibition of the expression of the *AGT* gene, such as inhibition of at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99%.

"Contact of a cell with the double-stranded RNAi agent" includes contacting a cell by any possible means. Contact of a cell with the double-stranded RNAi agent includes contacting a cell with this RNAi agent *in vitro* or contacting a cell with this RNAi agent *in vivo.* Such contact may be carried out directly or indirectly. Therefore, the RNAi agent may be, for example, physically contacted with the cell by an individual performing this method, or alternatively, the RNAi agent may be introduced into a circumstance that allows or causes it to subsequently contact the cell.

"Disease mediated by *AGT* expression" is intended to include any disease associated with the *AGT* gene or protein. Such a disease may be caused by, for example, overproduction of the AGT protein, mutation(s) in the *AGT* gene, abnormal cleavage of the AGT protein, or abnormal interactions between AGT and other proteins or other endogenous or exogenous substances.

A wide variety of entities can be coupled to the RNAi agents of the present application. The preferred moieties are ligands that are preferably covalently coupled directly or indirectly via an intervening tether.

Ligands may typically include therapeutic modifiers, *e.g.* for enhancing uptake; diagnostic compounds or reporter groups, *e.g.* for monitoring distribution; crosslinking agents; and moieties conferring nuclease resistance. General examples include lipids, steroids, vitamins, saccharides, proteins, peptides, polyamines, and peptidomimetics.

The ligand may include a naturally occurring substance, such as a protein (*e.g.* human serum albumin (HSA), low density lipoprotein (LDL), high density lipoprotein (HDL) or globulin); a carbohydrate (*e.g.* a dextran, amylopectin, chitin, chitosan, inulin, cyclodextrin or hyaluronic acid); or a lipid. The ligand may also be a recombinant or synthetic molecule, such as a synthetic polymer (*e.g.* a synthetic polyamino acid) and an oligonucleotide (*e.g.* an aptamer). Examples of polyamino acids include the following polyamino acids: polylysine (PLL), poly-L-aspartic acid, poly-L-glutamic acid, styrene-maleic anhydride copolymer, poly(L-lactide-co-glycolide) copolymer, divinyl ether-maleic anhydride copolymer, N-(2-hydroxypropyl)methacrylamide copolymer (HMPA), polyethylene glycol (PEG), polyvinyl alcohol (PVA), polyurethane, poly(2-ethylacrylic acid), N-isopropylacrylamide polymer, or polyphosphonazine. Examples of polyamines include: polyethyleneimine, polylysine (PLL), spermine, spermidine, polyamine, pseudopeptide-polyamine, peptidomimetic polyamine, dendritic polymer polyamine, arginine, amidine, protamine, cationic lipids, cationic porphyrins, quaternary salts of polyamines, or $\alpha$-helical peptides.

The ligand may also include a targeting group, such as a cell or tissue targeting agent that binds to a specified cell type (such as the kidney cell), *e.g.* lectins, glycoproteins, lipids or proteins, such as antibodies. The targeting group may be thyroid stimulating hormone, melanotropin, lectin, glycoprotein, surfactant protein A, mucin carbohydrate, polylactose, polygalactose, N-acetyl-galactosamine, N-acetyl-glucosamine polymannose, polytrehalose, glycosylated polyamino acid, polygalactose, transferrin, bisphosphonate, polyglutamate, polyaspartate, lipid, cholesterol, steroid, cholic acid, folate, vitamin B12, biotin, RGD peptide, RGD peptidomimetic, or aptamer.

Other examples of the ligand include dyes, intercalators (*e.g.* acridine), cross-linkers (*e.g.* psoralen and mitomycin C), porphyrins (TPPC4, texaphyrin, and Sapphyrin), polycyclic aromatic hydrocarbons (*e.g.* phenazine and dihydrophenazine), artificial endonucleases or chelators (*e.g.* EDTA), lipophilic molecules such as cholesterol, cholic acids, adamantane acetic acid, 1-pyrenebutyric acid, dihydrotestosterone, 1,3-bis-O(hexadecyl)glycerol, geranyloxyhexyl, cetylglycerol, borneol, menthol, 1,3-propanediol, heptadecanyl, palmitic acid, myristic acid, O3-(oleoyl)lithocholic acid, O3-(oleoyl)cholenic acid, dimethoxytriphenylmethyl, or phenoxazine-peptide conjugates (*e.g.* tentacledopeptide and Tat peptide), alkylating agents, phosphate esters, amino groups, thiol groups, PEG (*e.g.* PEG-40K), MPEG, $[MPEG]_2$, polyamino, alkyl, substituted alkyl, radiolabeled markers, enzymes, haptens (*e.g.* biotin), transport/absorption promoters (*e.g.* aspirin, vitamin E, and folic acid), synthetic ribonucleases (*e.g.* imidazole, diimidazole, histamine, imidazole clusters, acridine-imidazole conjugates, and $Eu^{3+}$ complexes of tetraaza macrocycles), dinitrophenyl, HRP, or AP.

The ligand may be a protein (such as a glycoprotein), or a peptide (such as a molecule having a specific affinity for a co-ligand), or an antibody (such as an antibody that binds to a specified cell type (such as a cancer cell, an endothelial cell, or a bone cell)). Ligands may also include hormones and hormone receptors. They may also include non-peptide species such as lipids, lectins, carbohydrates, vitamins, cofactors, multivalent lactose, multivalent galactose, N-acetyl-galactosamine, N-acetyl-glucosamine, multivalent mannose, multivalent fucose, or aptamers. The ligand may be, for example, lipopolysaccharide, an activator of p38MAP kinase, or an activator of NF-κB.

The ligand may be a substance, such as a drug, that can increase the uptake of iRNA agents into the cell, for example, by disrupting the cytoskeleton of the cell (*e.g.* by disrupting cellular microtubules, microfilaments and/or intermediate filaments). The drug may be, for example, taxon, vincristine, vinblastine, cytochalasin, nocodazole, japlakinolide, latrunculin A, phalloidin, swinholide A, indanocine, or myoservin.

The ligand may be any ligand capable of targeting a particular receptor. Examples of the ligand include folate, GalNAc, galactose, mannose, mannose-6P, glycoclusters (such as GalNAc clusters, mannose clusters, and galactose clusters), or an aptamer. A cluster is a combination of two or more saccharide units. These targeting ligands further include integrin receptor ligands, chemokine receptor ligands, transferrin, biotin, serotonin receptor ligands, PSMA, endothelin, GCPII, somatostatin, and LDL and HDL ligands. These ligands may also be nucleic acid-based ligands, such as an aptamer. This aptamer may be unmodified or possess any combination of modifications disclosed herein.

## BRIEF DESCRIPTION OF THE DRAWINGS

FIG. 1 shows the effects of DLR high-throughput screened candidate sequences on the *AGT* gene expression.
FIG. 2 shows the effects of Top10 candidate sequences on the *AGT* gene expression in Hep3B cells.
FIG. 3 shows the effects of Top10 modified candidate sequences on the *AGT* gene expression in Hep3B cells.
FIG. 4 shows the effects of candidate sequences on serum AGT protein in humanized mice.
FIG. 5 shows the effects of candidate sequences on the *AGT* gene mRNA at PHH level.
FIG. 6 shows the effects of candidate sequences on serum AGT protein in humanized mice.
FIG. 7 shows the effects of candidate sequences on serum AGT protein in rhesus monkeys.
FIG. 8 shows the effects of candidate sequences on serum AGT protein in hypertensive cynomolgus monkeys.

## DETAILED DESCRIPTION

### Example 1. Screening of AGT-siRNAs Activity

### I. Design of siRNAs

Based on the human *AGT* mRNA sequence, multiple AGT siRNAs were designed to target different sites. Each individual siRNA designed could target all transcripts of the target gene (as shown in Table 1). The above sequences (as shown in Table 2) were also checked for the minimal homology with other non-target gene sequence using the sequence alignment software. The sequence design was following the method published by Elbashir *et al.* 2002, Paddison *et al.* 2002, Reynolds *et al.* 2004 and Ui-Tei *et al.* 2004.

Table 1 Target Gene

| Target Gene | Species | Gene ID | NM_ID |
|---|---|---|---|
| AGT | *Homo sapiens* (human) | 183 | NM_001382817.3 |

Table 2 High-Throughout Screened Sequences

| DsRNA Name | SEQ ID NO: | Sequence (5'->3') | Length | Molecular Weight (g/mol) | GC Content |
|---|---|---|---|---|---|
| AGT36 | 40 | GUGCCUUGAUCUUGGACUUCC | 21 | 6602.95 | 52.4 |
| | 41 | GGAAGUCCAAGAUCAAGGCACUG | 23 | 7432.6 | 52.2 |
| AGT39 | 42 | CCUUGAUCUUGGACUUCCAGC | 21 | 6585.96 | 52.4 |
| | 43 | GCUGGAAGUCCAAGAUCAAGGCA | 23 | 7432.6 | 52.2 |
| AGT202 | 3 | GUCAUCCACAAUGAGAGUACC | 21 | 6679.11 | 47.6 |
| | 4 | GGUACUCUCAUUGUGGAUGACGA | 23 | 7364.47 | 47.8 |
| AGT203 | 1 | GUCAUCCACAAUGAGAGUACA | 21 | 6703.14 | 42.86 |
| | 2 | UGUACUCUCAUUGUGGAUGACGA | 23 | 7325.42 | 43.48 |
| AGT270 | 44 | CUUCAUACCUGCUCCAAUUCA | 21 | 6513.93 | 42.9 |
| | 45 | UGAAUUGGAGCAGGUAUGAAGGU | 23 | 7491.6 | 43.5 |
| AGT299 | 46 | CAUCCCCUGUGGAUGAAAAGG | 21 | 6735.14 | 52.4 |
| | 47 | CCUUUUCAUCCACAGGGGAUGUC | 23 | 7260.38 | 52.2 |
| AGT339 | 48 | GCUAGUCGCUGCAAAACUUGA | 21 | 6696.1 | 47.6 |
| | 49 | UCAAGUUUUGCAGCGACUAGCAC | 23 | 7307.45 | 47.8 |
| AGT343 | 50 | GUCGCUGCAAAACUUGACACC | 21 | 6655.08 | 52.4 |
| | 51 | GGUGUCAAGUUUUGCAGCGACUA | 23 | 7364.47 | 47.8 |
| AGT347 | 52 | CUGCAAAACUUGACACCGAAG | 21 | 6702.15 | 47.6 |
| | 53 | CUUCGGUGUCAAGUUUUGCAGCG | 23 | 7317.4 | 52.2 |
| AGT350 | 54 | CAAAACUUGACACCGAAGACA | 21 | 6709.19 | 42.9 |
| | 55 | UGUCUUCGGUGUCAAGUUUUGCA | 23 | 7279.35 | 43.5 |
| AGT412 | 56 | GGCUUCCGUAUAUAUGGCAUG | 21 | 6690.05 | 47.6 |
| | 57 | CAUGCCAUAUAUACGGAAGCCCA | 23 | 7313.5 | 47.8 |
| AGT413 | 58 | GCUUCCGUAUAUAUGGCAUGC | 21 | 6650.02 | 47.6 |
| | 59 | GCAUGCCAUAUAUACGGAAGCCC | 23 | 7329.5 | 52.2 |
| AGT562 | 60 | GUUCCUUGGAAGGACAAGAAC | 21 | 6759.17 | 47.6 |
| | 61 | GUUCUUGUCCUUCCAAGGAACAC | 23 | 7244.38 | 47.8 |
| AGT785 | 62 | CUCUGGACUUCACAGAACUGG | 21 | 6672.07 | 52.4 |
| | 63 | CCAGUUCUGUGAAGUCCAGAGAG | 23 | 7386.52 | 52.2 |
| AGT789 | 64 | GGACUUCACAGAACUGGAUGU | 21 | 6736.13 | 47.6 |
| | 65 | ACAUCCAGUUCUGUGAAGUCCAG | 23 | 7307.45 | 47.8 |
| AGT792 | 66 | CUUCACAGAACUGGAUGUUGC | 21 | 6673.06 | 47.6 |
| | 67 | GCAACAUCCAGUUCUGUGAAGUC | 23 | 7307.45 | 47.8 |
| AGT811 | 68 | GCUGCUGAGAAGAUUGACAGG | 21 | 6815.2 | 52.4 |
| | 69 | CCUGUCAAUCUUCUCAGCAGCAA | 23 | 7227.39 | 47.8 |
| AGT814 | 70 | GCUGAGAAGAUUGACAGGUUC | 21 | 6776.16 | 47.6 |
| | 71 | GAACCUGUCAAUCUUCUCAGCAG | 23 | 7267.42 | 47.8 |
| AGT815 | 72 | CUGAGAAGAUUGACAGGUUCA | 21 | 6760.16 | 42.9 |
| | 73 | UGAACCUGUCAAUCUUCUCAGCA | 23 | 7228.38 | 43.5 |

(continued)

| DsRNA Name | SEQ ID NO: | Sequence (5'->3') | Length | Molecular Weight (g/mol) | GC Content |
|---|---|---|---|---|---|
| AGT979 | 74 | GACAACAGCACCUCAGUGUCU | 21 | 6655.08 | 52.4 |
| | 75 | AGACACUGAGGUGCUGUUGUCCA | 23 | 7363.48 | 52.2 |
| AGT1040 | 76 | GUGACAUCCAGGACAACUUCU | 21 | 6656.07 | 47.6 |
| | 77 | AGAAGUUGUCCUGGAUGUCACUC | 23 | 7324.44 | 47.8 |
| AGT1144 | 78 | GGUCUCACUUUCCAGCAAAAC | 21 | 6616.04 | 47.6 |
| | 79 | GUUUUGCUGGAAAGUGAGACCCU | 23 | 7364.47 | 47.8 |
| AGT1145 | 80 | GUCUCACUUUCCAGCAAAACU | 21 | 6577 | 42.9 |
| | 81 | AGUUUUGCUGGAAAGUGAGACCC | 23 | 7387.51 | 47.8 |
| AGT1151 | 82 | CUUUCCAGCAAAACUCCCUCA | 21 | 6535.98 | 47.6 |
| | 83 | UGAGGGAGUUUUGCUGGAAAGUG | 23 | 7484.56 | 47.8 |
| AGT1156 | 84 | CAGCAAAACUCCCUCAACUGG | 21 | 6638.09 | 52.4 |
| | 85 | CCAGUUGAGGGAGUUUUGCUGGA | 23 | 7420.5 | 52.2 |
| AGT1224 | 86 | GGUGCUGCAAGGAUCUUAUGA | 21 | 6753.12 | 47.6 |
| | 87 | UCAUAAGAUCCUUGCAGCACCAG | 23 | 7290.46 | 47.8 |
| AGT1235 | 88 | GAUCUUAUGACCUGCAGGACC | 21 | 6672.07 | 52.4 |
| | 89 | GGUCCUGCAGGUCAUAAGAUCCU | 23 | 7323.45 | 52.2 |
| AGT1302 | 90 | CCUGCAAAAAUUGAGCAAUGA | 21 | 6727.17 | 38.1 |
| | 91 | UCAUUGCUCAAUUUUUGCAGGUU | 23 | 7224.31 | 34.8 |
| AGT1339 | 92 | GAGGUGCUGAACAGCAUUUUU | 21 | 6714.08 | 42.9 |
| | 93 | AAAAAUGCUGUUCAGCACCUCCC | 23 | 7250.43 | 47.8 |
| AGT1397 | 94 | CUACCCAACAGCUUAACAAGC | 21 | 6622.09 | 47.6 |
| | 95 | GCUUGUUAAGCUGUUGGGUAGAC | 23 | 7381.46 | 47.8 |
| AGT1401 | 96 | CCAACAGCUUAACAAGCCUGA | 21 | 6662.12 | 47.6 |
| | 97 | UCAGGCUUGUUAAGCUGUUGGGU | 23 | 7358.42 | 47.8 |
| AGT1447 | 98 | CCAUUCCUGUUUGCUGUGUAU | 21 | 6564.9 | 42.9 |
| | 99 | AUACACAGCAAACAGGAAUGGGC | 23 | 7439.64 | 47.8 |
| AGT1448 | 100 | CAUUCCUGUUUGCUGUGUAUG | 21 | 6604.93 | 42.9 |
| | 101 | CAUACACAGCAAACAGGAAUGGG | 23 | 7439.64 | 47.8 |
| AGT1452 | 102 | CCUGUUUGCUGUGUAUGAUCA | 21 | 6627.97 | 42.9 |
| | 103 | UGAUCAUACACAGCAAACAGGAA | 23 | 7384.6 | 39.1 |
| AGT1464 | 104 | GUAUGAUCAAAGCGCCACUGC | 21 | 6695.11 | 52.4 |
| | 105 | GCAGUGGCGCUUUGAUCAUACAC | 23 | 7323.45 | 52.2 |
| AGT1613 | 106 | GGACAAAUCAGCGAUGUGUCA | 21 | 6759.17 | 47.6 |
| | 107 | UGACACAUCGCUGAUUUGUCCGG | 23 | 7300.41 | 52.2 |
| AGT1638 | 108 | CAGUCUCCCACCUUUUCUUCU | 21 | 6443.82 | 47.6 |
| | 109 | AGAAGAAAAGGUGGGAGACUGGG | 23 | 7615.76 | 52.2 |
| AGT1642 | 110 | CUCCCACCUUUUCUUCUAAUG | 21 | 6467.85 | 42.9 |
| | 111 | CAUUAGAAGAAAAGGUGGGAGAC | 23 | 7520.69 | 43.5 |

(continued)

| DsRNA Name | SEQ ID NO: | Sequence (5'->3') | Length | Molecular Weight (g/mol) | GC Content |
|---|---|---|---|---|---|
| AGT1654 | 5 | CUUCUAAUGAGUCGACUUUGA | 21 | 6635.01 | 38.1 |
| | 6 | UCAAAGUCGACUCAUUAGAAGAA | 23 | 7362.55 | 34.8 |
| AGT1689 | 112 | GUUUCUCCUUGGUCUAAGUGU | 21 | 6604.93 | 42.9 |
| | 113 | ACACUUAGACCAAGGAGAAACGG | 23 | 7439.64 | 47.8 |
| AGT1714 | 114 | CAUGGAGUGAGCAGUAGAAGC | 21 | 6838.24 | 52.4 |
| | 115 | GCUUCUACUGCUCACUCCAUGCA | 23 | 7180.32 | 52.2 |
| AGT1758 | 116 | CCAGUUUGCUGGGUUUAUUUU | 21 | 6605.92 | 38.1 |
| | 117 | AAAAUAAACCCAGCAAACUGGGA | 23 | 7407.64 | 39.1 |
| AGT1765 | 118 | GCUGGGUUUAUUUUAGAGAAU | 21 | 6716.06 | 33.3 |
| | 119 | AUUCUCUAAAAUAAACCCAGCAA | 23 | 7266.49 | 30.4 |
| AGT1766 | 120 | CUGGGUUUAUUUUAGAGAAUG | 21 | 6716.06 | 33.3 |
| | 121 | CAUUCUCUAAAAUAAACCCAGCA | 23 | 7242.46 | 34.8 |
| AGT1795 | 122 | GAGGCAAGAACCAGUGUUUAG | 21 | 6799.2 | 47.6 |
| | 123 | CUAAACACUGGUUCUUGCCUCCC | 23 | 7180.32 | 52.2 |
| AGT1818 | 124 | CGGGACUACUGUUCCAAAAAG | 21 | 6719.14 | 47.6 |
| | 125 | CUUUUUGGAACAGUAGUCCCGCG | 23 | 7300.41 | 52.2 |
| AGT1821 | 126 | GACUACUGUUCCAAAAAGAAU | 21 | 6688.13 | 33.3 |
| | 127 | AUUCUUUUUGGAACAGUAGUCCC | 23 | 7246.36 | 39.1 |
| AGT1828 | 128 | GUUCCAAAAAGAAUUCCAACC | 21 | 6647.11 | 38.1 |
| | 129 | GGUUGGAAUUCUUUUUGGAACAG | 23 | 7366.45 | 39.1 |
| AGT1832 | 130 | CAAAAAGAAUUCCAACCGACC | 21 | 6669.16 | 42.9 |
| | 131 | GGUCGGUUGGAAUUCUUUUUGGA | 23 | 7359.41 | 43.5 |
| AGT1848 | 132 | CGACCAGCUUGUUUGUGAAAC | 21 | 6673.06 | 47.6 |
| | 133 | GUUUCACAAACAAGCUGGUCGGU | 23 | 7347.48 | 47.8 |
| AGT1851 | 134 | CCAGCUUGUUUGUGAAACAAA | 21 | 6681.09 | 38.1 |
| | 135 | UUUGUUUCACAAACAAGCUGGUC | 23 | 7269.4 | 39.1 |
| AGT1852 | 136 | CAGCUUGUUUGUGAAACAAAA | 21 | 6705.12 | 33.3 |
| | 137 | UUUUGUUUCACAAACAAGCUGGU | 23 | 7270.39 | 34.8 |
| AGT1854 | 138 | GCUUGUUUGUGAAACAAAAAA | 21 | 6729.15 | 28.6 |
| | 139 | UUUUUUGUUUCACAAACAAGCUG | 23 | 7231.35 | 30.4 |
| AGT1882 | 140 | CUUUUCAAGUUGAGAACAAAA | 21 | 6689.12 | 28.6 |
| | 141 | UUUUGUUCUCAACUUGAAAAGGG | 23 | 7310.42 | 34.8 |
| AGT1893 | 142 | GAGAACAAAAAUUGGGUUUUA | 21 | 6769.18 | 28.6 |
| | 143 | UAAAACCCAAUUUUUGUUCUCAA | 21 | 7198.36 | 26.1 |
| AGT1934 | 144 | GCAUUGCCUUCGGUUUGUAUU | 21 | 6604.93 | 42.9 |
| | 145 | AAUACAAACCGAAGGCAAUGCAA | 21 | 7407.64 | 39.1 |
| AGT1940 | 146 | CCUUCGGUUUGUAUUUAGUGU | 21 | 6605.92 | 38.1 |
| | 147 | ACACUAAAUACAAACCGAAGGCA | 21 | 7367.61 | 39.1 |

(continued)

| DsRNA Name | SEQ ID NO: | Sequence (5'->3') | Length | Molecular Weight (g/mol) | GC Content |
|---|---|---|---|---|---|
| AGT1944 | 148 | CGGUUUGUAUUUAGUGUCUUG | 21 | 6645.95 | 38.1 |
| | 149 | CAAGACACUAAAUACAAACCGAA | 21 | 7351.61 | 34.8 |
| AGT1945 | 150 | GGUUUGUAUUUAGUGUCUUGA | 21 | 6669.98 | 33.3 |
| | 151 | UCAAGACACUAAAUACAAACCGA | 21 | 7328.57 | 34.8 |
| AGT1961 | 152 | CUUGAAUGUAAGAACAUGACC | 21 | 6704.13 | 38.1 |
| | 153 | GGUCAUGUUCUUACAUUCAAGAC | 21 | 7269.4 | 39.1 |
| AGT1964 | 154 | GAAUGUAAGAACAUGACCUCC | 21 | 6703.14 | 42.9 |
| | 155 | GGAGGUCAUGUUCUUACAUUCAA | 21 | 7309.43 | 39.1 |
| AGT1968 | 156 | GUAAGAACAUGACCUCCGUGU | 21 | 6696.1 | 47.6 |
| | 157 | ACACGGAGGUCAUGUUCUUACAU | 21 | 7308.44 | 43.5 |
| AGT1990 | 158 | GUGUCUGUAAUACCUUAGUUU | 21 | 6612.96 | 33.3 |
| | 159 | AAACUAAGGUAUUACAGACACUA | 21 | 7346.55 | 30.4 |
| AGT1992 | 160 | GUCUGUAAUACCUUAGUUUUU | 21 | 6573.92 | 28.6 |
| | 161 | AAAAACUAAGGUAUUACAGACAC | 21 | 7369.59 | 30.4 |
| AGT2022 | 162 | GCUUGUGAUUUUUGAACAAUA | 21 | 6660.03 | 28.6 |
| | 163 | UAUUGUUCAAAAAUCACAAGCAU | 21 | 7284.47 | 26.1 |
| AGT2038 | 164 | CAAUACGUGAAAGAUGCAAGC | 21 | 6766.21 | 42.9 |
| | 165 | GCUUGCAUCUUUCACGUAUUGUU | 21 | 7200.28 | 39.1 |
| AGT2046 | 166 | GAAAGAUGCAAGCACCUGAAU | 21 | 6766.21 | 42.9 |
| | 167 | AUUCAGGUGCUUGCAUCUUUCAC | 21 | 7222.33 | 43.5 |
| AGT2050 | 168 | GAUGCAAGCACCUGAAUUUCU | 21 | 6657.06 | 42.9 |
| | 169 | AGAAAUUCAGGUGCUUGCAUCUU | 21 | 7309.43 | 39.1 |
| AGT2058 | 170 | CACCUGAAUUUCUGUUUGAAU | 21 | 6595.97 | 33.3 |
| | 171 | AUUCAAACAGAAAUUCAGGUGCU | 21 | 7339.51 | 34.8 |
| AGT2069 | 172 | CUGUUUGAAUGCGGAACCAUA | 21 | 6697.09 | 42.9 |
| | 173 | UAUGGUUCCGCAUUCAAACAGAA | 21 | 7315.48 | 39.1 |
| AGT2080 | 174 | CGGAACCAUAGCUGGUUAUUU | 21 | 6674.05 | 42.9 |
| | 175 | AAAUAACCAGCUAUGGUUCCGCA | 21 | 7314.49 | 43.5 |
| AGT2091 | 176 | CUGGUUAUUUCUCCCUUGUGU | 21 | 6541.86 | 42.9 |
| | 177 | ACACAAGGGAGAAAUAACCAGCU | 21 | 7423.64 | 43.5 |
| AGT2093 | 178 | GGUUAUUUCUCCCUUGUGUUA | 21 | 6565.89 | 38.1 |
| | 179 | UAACACAAGGGAGAAAUAACCAG | 21 | 7447.67 | 39.1 |
| AGT2103 | 180 | CCCUUGUGUUAGUAAUAAACG | 21 | 6658.05 | 38.1 |
| | 181 | CGUUUAUUACUAACACAAGGGAG | 21 | 7355.51 | 39.1 |
| AGT2108 | 182 | GUGUUAGUAAUAAACGUCUUG | 21 | 6699.07 | 33.3 |
| | 183 | CAAGACGUUUAUUACUAACACAA | 21 | 7283.48 | 30.4 |
| AGT2128 | 184 | GCCACAAUAAGCCUCCAAAAA | 21 | 6669.16 | 42.9 |
| | 185 | UUUUUGGAGGCUUAUUGUGGCAA | 21 | 7343.41 | 39.1 |

**II. Synthesis of siRNAs (natural RNA/2'-methoxy or 2'-fluoro-modified RNA/GalNAc-RNA)**

In the present application, oligonucleotides containing only ribonucleotides or only 2'-methoxy or 2'-fluoro modification(s) were synthesized at a theoretical yield of 1 μmol. All oligonucleotides were prepared on the LK-192X synthesizer using 1 μmol of universal Frit solid support (1000Å=100 nm, Biocomma) or GalNAc CPG (WuXi AppTec/Glen research, loading capacity: 30 umol/g). All phosphoramidite monomers (Shanghai Hongene Biotech/HitGen Inc.) were diluted in an anhydrous acetonitrile solvent at a ratio of 1:20 (g/mL) and coupled for 3 mins, repeated twice. 3% TCA was used for de-protection, 0.3 M benzylthiotetrazole acetonitrile solution was used for activation, and CAPA/CAPB and 50 mM $I_2$ solution were used for capping and oxidation, respectively. After Trityl-off synthesis, the solid support was transferred into a 2-mL centrifuge tube, and 1.2 mL of aqueous ammonia was added and heated in an oven at 65°C for 3 hrs to remove the protecting groups. Subsequently, the solution was cooled to room temperature and concentrated under vacuum for 30 mins. The solution was filtered through a 0.22-um filter membrane into a sample vial and subjected to single-strand purification by a semi-preparative reversed-phase purifier at an elution gradient of 7% to 30% (ACN:100 mM TEAA) for 10 min at a flow rate of 5 mL/min. After purification, the solution was concentrated under vacuum and dried via rotary evaporation at room temperature. Finally, the sample was dissolved in water, and the solution was desalted on a GE Hi-Trap desalting column to elute the final oligonucleotide product. The property and purity of the oligonucleotide were confirmed using ESI-MS and IEX HPLC, respectively. The concentration was measured by UV in the microplate reader. Equal molar amount of the sense strand and the antisense strand were mixed in a new delivery tube, heated at 95°C for 5 mins and slowly cooled down to room temperature for double stand annealing, and dried via rotary evaporation at room temperature using a vacuum concentrator to get the final product.

**III. Inhibitory activity assay of *AGT* siRNAs in the *in vitro* psicheck-2 system**

1. Plasmid constructionfor assay

Psicheck-2 was used to construct the recombinant plasmid (Generay Biotech (Shanghai) Co., Ltd.) containing the target sequences of all *AGT* siRNAs to be tested, and the cloning sites were at 5'XhoI and 3'NotI of psicheck-2.

2. Co-transfection of *AGT* siRNAs and the recombinant plasmids into different cells

All cells were obtained from National Collection of Authenticated Cell Cultures or from other publicly available sources; and all the other reagents were commercially available.

Table 3 Cell Name and Type

| Cell Name | 293T | Hep3B |
|---|---|---|
| Cell Type | Human renal epithelial cell | Human liver cancer cell |

Cells were cultured in DMEM medium containing 10% fetal bovine serum in the 37°C incubator, 5% $CO_2$. Transfection was performed when the cells were viable and in the logarithmic growth phase (70% confluence). The cell density was adjusted, and the cells were seeded into a 24-well plate at $1.5\times10^5$ cells per well. Transfection system was prepared: 250 μL of Opti-MEM, 40 ng of recombinant plasmid, and 5 μL of 10 nM siRNA were mixed; 250 μL of Opti-MEM and 2.5 μL of Lipofectamine 2000 transfection reagent were mixed, incubated for 5 mins; these two mixtures were then mixed and incubated for 20 mins. This transfection system was added to the 24-well plate and incubated in the 37°C incubator, 5% $CO_2$ for 6 hrs. The supernatant was then aspirated, and 1 mL of complete medium was added to each well. The cells were further cultured for 24 hrs.

In addition to the experimental groups, the following control groups were also included for each run of cell transfection: NC (irrelevant siRNA) as the negative control group and Lipofectamine as the transfection reagent control group. Three replicates were included for each experimental group and control group. The nucleotide sequence of the NC group was as follows:

sense strand: UUCUCCGAACGUGUCACGUTT (SEQ ID NO: 186);
antisense strand: ACGUGACACGUUCGGAGAATT (SEQ ID NO: 187).

3. DLR assay

The assay was carried out using Dual-Luciferase Reporter Assay System (Promega). The treated cells were

collected and lysed according to the instructions of the kit. The Infinite Eplex microplate reader (TECAN) was used to detect the fluorescence intensities of the *Photinus pyralis* luciferase and the *Renilla reniformis* luciferase sequentially, and the intensity ratio of the *Renilla reniformis* luciferase to the *Photinus pyralis* luciferase was calculated and normalized to the NC group. The nucleotide sequence of the positive control AD85481 was as follows:

sense strand: GUCAUCCACAAUGAGAGUACA (SEQ ID NO: 188);
antisense strand: UGUACUCUCAUUGUGGAUGACGA (SEQ ID NO: 189).

Table 4 DLR Detection Results

| No. | Mean |
|---|---|
| Lipo2000 | 0.924565 |
| NC | 1 |
| AD85481 | 0.331757 |
| AGT202 | 0.239385 |
| AGT270 | 0.220842 |
| AGT339 | 0.15651 |
| AGT343 | 0.24037 |
| AGT347 | 0.232444 |
| AGT350 | 0.354286 |
| AGT412 | 0.243721 |
| AGT413 | 0.330309 |
| AGT814 | 0.348109 |
| AGT815 | 0.229583 |
| AGT1144 | 0.294227 |
| AGT1145 | 0.227198 |
| AGT1151 | 0.161185 |
| AGT1156 | 0.224591 |
| AGT1224 | 0.290155 |
| AGT1613 | 0.288361 |
| AGT1638 | 0.293461 |
| AGT1642 | 0.279215 |
| AGT1654 | 0.19138 |
| AGT1689 | 0.297011 |
| AGT1828 | 0.263628 |
| AGT1832 | 0.261416 |
| AGT1854 | 0.372858 |
| AGT1990 | 0.264215 |
| AGT1992 | 0.355199 |
| AGT2022 | 0.28283 |
| AGT2093 | 0.234824 |
| AGT2103 | 0.215051 |
| AGT2108 | 0.228507 |

(continued)

| No. | Mean |
|---|---|
| AGT2128 | 0.203673 |

As shown in FIG. 1, through DLR screening of the *AGT* siRNAs in 293T cells, top 30 siRNA molecules with a higher cellular activity were identified based on the relative activity of luciferase. Table 1 DLR assay results showing the means of the dual luciferase reporter expression levels in the *AGT* siRNA experimental groups relative to the NC group.

## IV. Screening of *AGT* siRNAs by qPCR

1. Transfection of *AGT* siRNAs in Hep3B cells

Hep3B cells were cultured in DMEM medium containing 10% fetal bovine serum in the 37°C incubator, 5% $CO_2$. Transfection was performed when the cells were viable and in the logarithmic growth phase (70% confluence). The cell density was adjusted, and the cells were seeded into a 24-well plate at $1.5\times10^5$ cells per well. Transfection system was prepared: 250 μL of Opti-MEM and 5 μL of 10 nM siRNA were mixed; 250 μL of Opti-MEM and 2.5 μL of Lipofectamine 2000 transfection reagent were mixed and incubated for 5 mins; these two mixtures were then mixed and incubated for 20 mins. This transfection system was added to the 24-well plate and incubated in the 37°C incubator, 5% $CO_2$ for 6 hrs. The supernatant was aspirated, and 1 mL of complete medium was added to each well. The cells were further cultured for 24 hrs. In addition to the experimental groups, the following control groups were also included for each run of cell transfection: the NC group as the negative control group (irrelevant siRNA) and the Lipofectamine group as the transfection reagent control group (without siRNA).

2. Quantitative real-time PCR (qRT-PCR) assay

The cells were lysed 24 hrs after transfection, and the total RNAs were extracted from the cells using the column extraction kit (Vazyme). β-actin was incorporated as the reference gene, the qRT-PCR reaction was carried out on the CFX96 fluorescence quantitative PCR system (Bio-Rad) following the Taqman method. The primers were listed in the table below.

Table 5 Primer Sequence Information

| Primer Name | Sequence (5' to 3') | SEQ ID NO: |
|---|---|---|
| H-GAPDH-F | GGTCGGAGTCAACGGATTT | 190 |
| H-GAPDH-R | CCAGCATCGCCCCACTTGA | 191 |
| H-AGT-F | TCCAGCCTCACTATGCCTCT | 192 |
| H-AGT-R | GGCTCTCTCTCATCCGCTTC | 193 |

3. Data analysis

After the PCR reaction, relative quantification was carried out following the 2-ΔΔCt (Livak) method using the reference gene as a standard. The results in Table 6 and FIG. 2 showed that 10 siRNA sequences achieved the superior potency of *AGT* mRNA reduction compared to the positive control AD85481, out of the top 30 siRNAs selected by DLR assay.

Table 6 Inhibitory Activities of Top 10 siRNA Sequences

| No. | Relative Expression Level |
|---|---|
| Lipo2000 | 0.87362 |
| NC | 1 |
| AD85481 | 0.57457 |
| AGT202 | 0.27193 |
| AGT814 | 0.290318 |

(continued)

| No. | Relative Expression Level |
|---|---|
| AGT815 | 0.164974 |
| AGT1452 | 0.219945 |
| AGT1613 | 0.124849 |
| AGT1638 | 0.220761 |
| AGT1642 | 0.261598 |
| AGT1654 | 0.150278 |
| AGT1689 | 0.238988 |
| AGT1854 | 0.27335 |

**Example 2. Optimization of *AGT* siRNAs**

**I*. Assay on inhibitory activity* of modified siRNA on *ANGPTL3* gene**

The hit siRNA sequences obtained by the previous screenings were modified respectively (Table 7). A combination of fluoro and methoxy modifications was introduced at different positions of the sense strand and the antisense strand, the antisense strand was modified with methoxy wherever possible. The steps of siRNA synthesis, transfection in the Hep3B cells, and qRT-PCR assay of the modified sequences were the same as described in Example 1. The transfection concentration was 0.1 nM.

Table 7 Top 11 Highly Active modified siRNA Sequences

| Name | | Sequence (5'->3') |
|---|---|---|
| AD85481-E-G | Sense strand | GmsUmsCmAmUmCmCfAmCfAfAfUmGmAmGmAmGmUmAmCmAm-L96 (SEQ ID NO: 29) |
| | Antisense strand | UmsGfsUmAmCm(Tgn)CmUmCmAmUmUmGmUfGmGfAmUmGmAmCmsGmsAm (SEQ ID NO: 194) |
| AGT202-E-G | Sense strand | GmsUmsCmAmUmCmCfAmCfAfAfUmGmAmGmAmGmUmAmCmCm- L96 (SEQ ID NO: 30) |
| | Antisense strand | GmsGfsUmAmCmUmCmUmCmAmUmUmGmUfGmGfAmUmGmAmCmsGmsAm (SEQ ID NO: 9) |
| AGT814-E-G | Sense strand | GmsCmsUmGmAmGmAfAmGfAfUfUmGmAmCmAmGmGmUmUmCm- L96 (SEQ ID NO: 31) |
| | Antisense strand | GmsAfsAmCmCmUmGmUmCmAmAmUmCmUfUmCfUmCmAmGmCmsAmsGm (SEQ ID NO: 11) |
| AGT815-E-G | Sense strand | CmsUmsGmAmGmAmAfGmAfUfUfGmAmCmAmGmGmUmUmCmAm- L96 (SEQ ID NO: 32) |
| | Antisense strand | UmsGfsAmAmCmCmUmGmUmCmAmAmUmCfUmUfCmUmCmAmGmsCmsAm (SEQ ID NO: 13) |
| AGT1452-E-G | Sense strand | CmsCmsUmGmUmUmUfGmCfUfGfUmGmUmAmUmGmAmUmCmAm- L96 (SEQ ID NO: 33) |
| | Antisense strand | UmsGfsAmUmCmAmUmAmCmAmCmAmGmCfAmAfAmCmAmGmGmsAmsAm (SEQ ID NO: 15) |
| AGT1613-E-G | Sense strand | GmsGmsAmCmAmAmAfUmCfAfGfCmGmAmUmGmUmGmUmCmAm- L96 (SEQ ID NO: 34) |
| | Antisense strand | UmsGfsAmCmAmCmAmUmCmGmCmUmGmAfUmUfUmGmUmCmCmsGmsGm (SEQ ID NO: 17) |

(continued)

| Name | | Sequence (5'->3') |
|---|---|---|
| AGT1638-E-G | Sense strand | CmsAmsGmUmCmUmCfCmCfAfCfCmUmUmUmCmUmUmCmUm- L96 (SEQ ID NO: 35) |
| | Antisense strand | AmsGfsAmAmGmAmAmAmAmGmGmUmGmGfGmAfGmAmCmUmGmsGmsGm (SEQ ID NO: 19) |
| AGT1642-E-G | Sense strand | CmsUmsCmCmCmAmCfCmUfUfUfUmCmUmUmCmUmAmAmUmGm- L96 (SEQ ID NO: 36) |
| | Antisense strand | CmsAfsUmUmAmGmAmAmGmAmAmAmAmGfGmUfGmGmGmAmGmsAmsCm (SEQ ID NO: 21) |
| AGT1654-E-G | Sense strand | CmsUmsUmCmUmAmAfUmGfAfGfUmCmGmAmCmUmUmUmGmAm- L96 (SEQ ID NO: 37) |
| | Antisense strand | UmsCfsAmAmAmGmUmCmGmAmCmUmCmAfUmUfAmGmAmAmGmsAmsAm (SEQ ID NO: 23) |
| AGT1689-E-G | Sense strand | GmsUmsUmUmCmUmCfCmUfUfGfGmUmCmUmAmAmGmUmGmUm- L96 (SEQ ID NO: 38) |
| | Antisense strand | AmsCfsAmCmUmUmAmGmAmCmCmAmAmGfGmAfGmAmAmAmCmsGmsGm (SEQ ID NO: 25) |
| AGT1854-E-G | Sense strand | GmsCmsUmUmGmUmUfUmGfUfGfAmAmAmCmAmAmAmAmAmAm- L96 (SEQ ID NO: 39) |
| | Antisense strand | UmsUfsUmUmUmUmGmUmUmUmCmAmCmAfAmAfCmAmAmGmCmsUmsGm (SEQ ID NO: 27) |
| AGT203-E-G | Sense strand | GmsUmsCmAmUmCmCfAmCfAfAfUmGmAmGmAmGmUmAmCmAm-L96 (SEQ ID NO: 29) |
| | Antisense strand | UmsGfsUmAmCmUfCmUmCmAmUmUmGmUfGmGfAmUmGmAmCmsGmsAm (SEQ ID NO: 7) |
| Note: Am, Um, Cm and Gm represented 2'-O-methyl-modified ribonucleotides A, U, C and G, respectively; Af, Uf, Cf and Gf represented 2'-fluoro-modified ribonucleotides A, U, C and G, respectively; (s) represented the two adjacent nucleotides were linked via a phosphorothioate backbone, and (Tgn) represented (s)-GNA-T with a specific chemical formula shown below. | | |

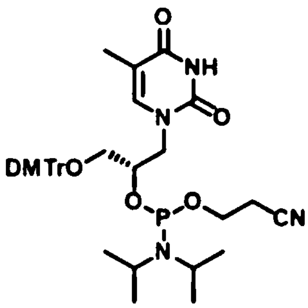

*(S)*-GNA-T

As shown in FIG. 3, the modification protocol was crucial as some modifications reduced the inhibitory activity. The optimal modification pattern resulted in a high inhibitory activity: (1) the antisense strand had an overhang with a 5'(s) mN(s)mN3' structure at the 3'-terminus, which contributed to the loading of the antisense strand to RISC and enhanced the interference activity of RNAi without compromising the stability; (2) the antisense strand comprised a fluoro modification at positions 2, 14 and 16 at least from the 5'-terminus, and the methoxy modification at the remaining positions wherever possible, which facilitated its binding to RISC; (3) the antisense strand had two phosphorothioate modifications at least from the 3'-terminus and the 5'-terminus, which stabilized the nucleic acid; (4) the sense strand comprised consecutive fluoro modifications at positions 7 and 9 to 11 from the 5'-terminus, and the methoxy modification at the remaining positions

wherever possible, which promoted its binding to RISC; and (5) the sense strand comprised two phosphorothioate modifications at least from the 5'-terminus, and GalNAc covalently coupled to the 3'-terminus. In the 5'(s)mN(s)mN3' structure, N represented any nucleotide, m at the right side of N represented the nucleotide was 2'-O-methyl-modified, and (s) represented the two adjacent nucleotides were linked via a phosphorothioate backbone. The results in Table 8 indicated that among 11 hit siRNA sequences, AGT202-E-G, AGT814-EG, AGT1613-E-G, AGT1638-E-G, AGT1642-E-G, AGT1654-E-G, AGT1689-E-G, and AGT203-E-G exhibited a comparable inhibitory activity in the Hep3B cells relative to the positive control AD85481 at a transfection concentration of 0.1 nM, and all of them achieved 90% or more *AGT* mRNA knockdown.

Table 8 Inhibitory Activities of Top 8 Modified siRNA Sequences

| No. | Relative Expression Level |
|---|---|
| NC | 1 |
| AD85481-E-G | 0.026 |
| AGT202-E-G | 0.023 |
| AGT814-E-G | 0.037 |
| AGT1613-E-G | 0.080 |
| AGT1638-E-G | 0.042 |
| AGT1642-E-G | 0.039 |
| AGT1654-E-G | 0.0150 |
| AGT1689-E-G | 0.043 |
| AGT203-E-G | 0.073 |

**Example *3. In Vivo* Efficacy in Mice**

In this experiment, 72 mix-gender 6-8 weeks old humanized *AGT* mice (GemPharmatech Co., Ltd.) were randomly grouped according to the body weight, and 8 mice (4 females and 4 males) were included in each group. The mice were subcutaneously injected with a single dose of 3 mg/kg AGT1654-E-G, AGT202-E-G, AGT1638-E-G, AGT814-E-G, AGT1613-E-G, AGT1642-E-G, AGT1689-E-G or AGT203-E-G. Blood was collected on days -3, 7, 14, 21, and 28 (fasting for 4 to 5 hrs before each blood collection, with the dosing day as Day 0). Serum was separated from the blood samples. The expression level of AGT protein in the serum was measured using Human Total Angiotensinogen Assay Kit (IBL, #27412) for the intergroup comparison.

Table 9 Average AGT Protein Level in Example 3 (normalized to pre-treatment AGT level)

| | Day 7 | Day 14 | Day 21 | Day 28 |
|---|---|---|---|---|
| AGT202-E-G | 0.359 | 0.289 | 0.304 | 0.364 |
| AGT1638-E-G | 0.264 | 0.267 | 0.296 | 0.320 |
| AGT1654-E-G | 0.199 | 0.161 | 0.191 | 0.205 |
| AGT203-E-G | 0.20 | 0.18 | 0.17 | 0.22 |

The pharmacodynamic results in mice showed (FIG. 4, Table 9) that AGT1654-E-G, AGT202-E-G, AGT1638-E-G, and AGT203-E-G lowered the serum AGT level by approximately 70%-80% or more, with a sustained effect for at least 28 days.

**Example 4. Pharmacodynamic Effect of siRNA in the Human Primary Hepatocytes (PHHs)**

4.1 The compound were internalized into the human primary hepatocytes via free uptake. The detailed process was described as follows:

1) The compound was diluted with nuclease-free water to 10 times the final concentration.
2) Two vials of cryopreserved cells were taken out of the liquid nitrogen tank, and gently swirled in a water bath until the

freezing medium was completely thawed. The cells were transferred into a culture medium containing 10% fetal bovine serum. The cell density was adjusted to $6.7\times10^5$ cells/mL.
3) 25 μL of the compound was measured into a cell plate and 225 μL of the cell suspension obtained in step 2) was added. The final compound concentrations were 100 nM and 500 nM, with 3 replicates for each concentration. Blank was a control group containing cells treated with nuclease-free water.

The above experiment was repeated three times.

**4.2 Fluorescence qRT-PCR**

1) The total RNAs purified from the cells were proceeded to qRT-PCR reaction according to the method described in the instruction of the HiScript III RT SuperMix (+gDNA wiper) kit. The commercially available primers and probes were purchased from Thermo Fisher Scientific (Catalog #4331182). The qRT-PCR reaction system was prepared as shown in the table below.

Table 10 qRT-PCR Reaction System

| Components | Content (1 reaction) |
|---|---|
| TaqMan® Assays and Arrays | 1/6 μL |
| FastStart Universal Probe Master | 5 μL |
| cDNA | 2 μL |
| RNase free water | 17/6 μL |
| Total vol. (μL) | 10 μL |

2) GAPDH was used as the reference gene. The expression level of the target gene RNA in each sample was calculated based on the relative quantitative method ΔΔCt. The relative expression level of the target gene was presented as 2-ΔΔCT.
3) The qRT-PCR protocol was as follows: the reaction was carried out at 95°C for 10 min, then 95°C for 15 seconds, and 60°C for 1 min for 40 cycles.
4) Data analysis

$$\Delta CT = \text{average Ct value of target gene} - \text{average Ct value of reference gene}$$

$$\Delta\Delta CT = \Delta CT\ \text{(sample group)} - \Delta CT\ \text{(nuclease-free water control group)};$$

$$\text{Relative mRNA expression level of target gene} = 2^{-\Delta\Delta CT}$$

Percentage inhibition (%) = (1 - relative expression level of sample / average relative expression level in control group) × 100

**4.3 Data analysis**

Table 11 Inhibition Rate of siRNA against *AGT* Gene mRNA in PHH

| Cell | siRNA | N=1 | | N=2 | | N=3 | |
|---|---|---|---|---|---|---|---|
| | | Average inhibition rate % | | Average inhibition rate % | | Average inhibition rate % | |
| | | 500 nM | 100 nM | 500 nM | 100 nM | 500 nM | 100 nM |
| PHH (lot:BXU) | AGT202-E-G | 78.42 | 75.84 | 72.12 | 72.19 | 72.43 | 74.88 |
| | AGT203-E-G | 95.16 | 94.23 | 95.78 | 95.07 | 95.05 | 94.83 |
| | AD85481-E-G | 95.90 | 94.78 | 95.41 | 95.64 | 95.13 | 95.80 |

The PHH uptake experiment demonstrated the endocytosis of siRNA into the liver and its knockdown efficiency. The results (Table 11, FIG. 5) showed that AGT203-E-G and AD85481-E-G, achieved a robust inhibitory effect against the *AGT* gene mRNA with a ratio of 90% or more at the concentration of 100 nM and 500 nM.

**Example 5. Assay on *In Vivo* Efficacy in Mice**

In this experiment, 40 male 6-8 weeks old humanized *AGT* mice (GemPharmatech Co., Ltd.) were randomly grouped according to the body weight. 10 mice were included in each group. The mice were subcutaneously injected with a single dose of 3 mg/kg normal saline, AGT202-E-G, AD85481-E-G or AGT203-E-G. Blood was collected on days -3, 7, 14, 21, and 28 (fasting for 4 to 5 hours before each blood collection, with the dosing day as Day 0). Serum was separated from the blood samples. The expression levels of AGT protein in the serum was measured using Human Total Angiotensinogen Assay Kit (IBL, #27412) for the intergroup comparison.

Table 12 Average AGT protein level in Example 5 (normalized to pre-treatment AGT level)

| Time | AGT202-E-G | AGT203-E-G | AD85481-E-G |
|---|---|---|---|
| Day 7 | 0.36 | 0.08 | 0.09 |
| Day 14 | 0.31 | 0.07 | 0.08 |
| Day 21 | 0.26 | 0.06 | 0.09 |
| Day 28 | 0.33 | 0.09 | 0.15 |
| Day 35 | 0.42 | 0.10 | 0.18 |
| Day 42 | 0.43 | 0.13 | 0.26 |
| Day 49 | 0.57 | 0.19 | 0.35 |
| Day 56 | 0.58 | 0.26 | 0.45 |
| Day 63 | 0.65 | 0.32 | 0.55 |
| Day 70 | 0.66 | 0.39 | 0.57 |
| Day 77 | 0.71 | 0.43 | 0.69 |
| Day 84 | 0.75 | 0.56 | 0.79 |
| Day 91 | 0.88 | 0.63 | 0.80 |
| Day 98 | 0.83 | 0.74 | 0.89 |

The pharmacodynamic results in mice showed (FIG. 6, Table 12) that both AD85481-E-G and AGT203-E-G reduced the serum AGT level to the lowest point on Day 14 post dosing, with a rate of 90% or more, and a sustained effect was also observed. Compared to AD85481-E-G, AGT202-E-G exhibited an even longer duration of action.

**Example 6. *In Vivo* Efficacy in Rhesus Monkeys**

In this experiment, 8 rhesus monkeys (JOINN Biologics) were randomly allocated into 4 groups, including 3 experimental groups and a positive control group (4 test articles: AD85481-E-G, AGT202-E-G, AGT1638-E-G and AGT1654-E-G), according to the body weight. There were 2 animals (1 female and 1 male) in each group. The animals were subcutaneously injected with one of the test articles at a single dose of 3 mg/kg. Blood was collected from forelimb

cephalic vein on days -1, 4, 7, 11, 14, 18, 21, 28, 32, 35, 39, 42, 49, and 56 (fasting overnight before each blood collection, with the dosing day as Day 0). The serum was separated from the blood samples. The expression levels of AGT protein in the serum was measured using Human Total Angiotensinogen Assay Kit (IBL, #27412) for the intergroup comparison.

Table 13 Average AGT protein Average AGT protein level in Example 6

| (Normalized to pre-treatment AGT level) | | | |
|---|---|---|---|
| | AD85481-E-G | AGT202 E-G | AGT1638 E-G |
| Day 4 | 0.703 | 0.879 | 0.657 |
| Day 7 | 0.382 | 0.401 | 0.335 |
| Day 11 | 0.275 | 0.208 | 0.198 |
| Day 14 | 0.381 | 0.172 | 0.201 |
| Day 18 | 0.250 | 0.115 | 0.184 |
| Day 21 | 0.240 | 0.101 | 0.169 |
| Day 28 | 0.253 | 0.056 | 0.160 |
| Day 32 | 0.205 | 0.061 | 0.148 |
| Day 35 | 0.208 | 0.061 | 0.169 |
| Day 39 | 0.204 | 0.065 | 0.177 |
| Day 42 | 0.204 | 0.059 | 0.197 |
| Day 49 | 0.227 | 0.084 | 0.254 |
| Day 56 | 0.315 | 0.091 | 0.260 |
| Day 63 | 0.286 | 0.096 | 0.269 |

The results (FIG. 7, Table 13) indicated that AGT202-E-G achieved a robust and sustained effect on the serum AGT protein reduction compared to the baseline level, with a rate of approximately 80% on Day 11 post dosing, 90% or more on Day 28, and 90% serum AGT protein reduction was maintained until Day 63; AGT1638-E-G also achieved a robust and sustained effect on the serum AGT protein reduction, with a rate of approximately 80% on Day 11, and 73% reduction on the serum AGT protein level was maintained until Day 63; On the contrary, the positive control AD85481-E-G did not achieve 80% serum AGT protein reduction until Day 32, and 80% reduction was maintained for about 10 days and began to rebound. In conclusion, AGT202-E-G and AGT1638-E-G demonstrated significantly better efficacy and longer duration of action compared to the positive control AD85481-E-G.

**Example 7. *In Vivo* Efficacy in Hypertension Cynomolgus Monkey Model**

In this experiment, 6 spontaneous hypertension cynomolgus monkeys (Kunming Biomed International), with a systolic blood pressure (SBP) equal to or higher than 140 mmHg and a diastolic blood pressure (DBP) equal to or higher than 70 mmHg, were used. They were randomly assigned to the AGT203 E-G low-dose group (1.5 mg/kg) and the AGT203 E-G high-dose group (3 mg/kg) according to the body weight. 3 animals were included in each group. The animals were subcutaneously injected with a single dose of the test article. Blood was collected from peripheral veins on days -9, -2, 4, 7, 14, 21, 28, 42, and 56 (fasting overnight before each blood collection, with the dosing day as Day 0). The serum was separated from the blood samples for AGT protein assay. In addition, on days -7, 23 and 54, the SBP and DBP were measured by a high-resolution oscilloscope (HDO) device at the left forelimb wrist or the base of the tail of each animal, and the mean arterial pressure (MAP) was calculated.

Table 14 Average AGT protein Average AGT protein level in Example 7

| (Normalized to pre -treatment AGT level) | | | | | | | |
|---|---|---|---|---|---|---|---|
| | Day 4 | Day 7 | Day 14 | Day 21 | Day 28 | Day 42 | Day 56 |
| AGT203 E-G (1.5 mg/kg) | 0.385 | 0.179 | 0.042 | 0.026 | 0.012 | 0.012 | 0.019 |
| AGT203 E-G (3 mg/kg) | 0.337 | 0.174 | 0.028 | 0.033 | 0.024 | 0.021 | 0.016 |

Table 15 Blood Pressure (BP) and Mean AGT Protein Reduction in Example 7

| (Normalized to pre-treatment AGT level) | | | | | | | | | |
|---|---|---|---|---|---|---|---|---|---|
| BP %Change Results | SBP (%) | | | DBP (%) | | | MAP (%) | | |
| | Day -7 | Day 23 | Day 54 | Day -7 | Day 23 | Day 54 | Day -7 | Day 23 | Day 54 |
| AGT203 E-G (1.5 mg/kg) | 0.0 | -12.4 | -16.6 | 0.0 | -13.3 | -19.1 | 0.0 | -13.0 | -17.9 |
| AGT203 E-G (3 mg/kg) | 0.0 | -12.5 | -16.1 | 0.0 | -19.9 | -30.2 | 0.0 | -15.9 | -23.1 |

The results (FIG. 8, Tables 14-15) indicated that, both the AGT203 E-G high-dose group and the low-dose group achieved a sustained effect on the serum AGT protein reduction compared to the baseline level before dosing, with a rate of 90% or more on Day 14, and the serum AGT protein level was maintained at a low level until Day 56. On Day 54 post dosing, SBP and DBP were reduced by 16.6% and 19.1%, respectively in the AGT203 E-G low-dose group, and SBP and DBP were reduced by 16.1% and 30.2%, respectively in the AGT203 E-G high-dose group on Day 54. The above results demonstrated that a remarkable efficacy of hypertension alleviation with a significantly reduced AGT protein level was achieved by the treatment of the AGT203 E-G. As shown in FIG. 7 and FIG. 8, AGT203-E-G reduced the serum AGT protein to a lower level more rapidly and maintained the low level more persistently compared to AGT202-E-G and AGT1638-E-G, and AGT203-E-G exhibited a better *AGT* mRNA inhibition potency compared to AGT202-E-G and AGT1638-E-G.

## Claims

1. A double-stranded RNAi agent, comprising a sense strand and an antisense strand, wherein nucleotide sequences of the sense strand and the antisense strand are as set forth in any one of dsRNA sequences in Table 2.

2. The double-stranded RNAi agent according to claim 1, comprising a sense strand and an antisense strand, wherein

   (1) the sense strand comprises the nucleotide sequence GUCAUCCACAAUGAGAGUACA (SEQ ID NO: 1) (5' to 3' direction) and/or the antisense strand comprises the nucleotide sequence UGUACUCUCAUUGUGGAUGACGA (SEQ ID NO: 2) (5' to 3' direction);
   (2) the sense strand comprises the nucleotide sequence GUCAUCCACAAUGAGAGUACC (SEQ ID NO: 3) (5' to 3' direction) and/or the antisense strand comprises the nucleotide sequence GGUACUCUCAUUGUGGAUGACGA (SEQ ID NO: 4) (5' to 3' direction); or
   (3) the sense strand comprises the nucleotide sequence CUUCUAAUGAGUCGACUUUGA (SEQ ID NO: 5) (5' to 3' direction) and/or the antisense strand comprises the nucleotide sequence UCAAAGUCGACUCAUUAGAAGAA (SEQ ID NO: 6) (5' to 3' direction).

3. The double-stranded RNAi agent according to claim 1 or 2, wherein the double-stranded RNAi agent comprises, on one or more nucleotides of the sense strand and the antisense strand thereof, one or more modifications selected from the group consisting of: 2'-methoxyethyl, 2'-O-alkyl, 2'-O-allyl, 2'-C-allyl, 2'-fluoro, 2'-deoxy, 2'-hydroxy, a locked nucleic acid modification, a ring-opened or unlocked nucleic acid modification, a DNA modification, and a fluorescent probe modification; preferably, both the sense strand and the antisense strand of the double-stranded RNAi agent contain 2'-O-methyl and/or 2'-fluoro modifications.

4. The double-stranded RNAi agent according to claim 3, wherein the double-stranded RNAi agent further comprises at least one phosphorothioate or methylphosphonate internucleotide linkage, preferably at least one phosphorothioate linkage; further preferably, the internucleotide linkage is located at 5'-terminus and 3'-terminus of the sense strand and antisense strand; and more preferably, the internucleotide linkage is located within three nucleotides at the 5'-terminus and the 3'-terminus of the sense strand and the antisense strand.

5. The double-stranded RNAi agent according to any one of claims 1 to 4, wherein the double-stranded RNAi agent comprises: (1) the antisense strand having an overhang with a 5'(s)mN(s)mN3' structure at the 3'-terminus; (2) the antisense strand comprising a fluoro modification at least at positions 2, 14 and 16 from the 5'-terminus, and a methoxy modification at remaining positions; (3) the antisense strand comprising at least two phosphorothioate modifications from the 3'-terminus and the 5'-terminus, preferably the antisense strand comprising a phosphorothioate backbone modification in at least the first and second internucleotide linkages from the 3'-terminus and the 5'-terminus; (4) the

sense strand comprising a fluoro modification at positions 7 and 9 to 11 from the 5'-terminus, and a methoxy modification at remaining positions; and (5) the sense strand comprising at least two phosphorothioate modifications from the 5'-terminus, preferably the sense strand comprising a phosphorothioate backbone modification in at least the first and second internucleotide linkages from the 5'-terminus.

**6.** The double-stranded RNAi agent according to any one of claims 1 to 5, wherein the double-stranded RNAi agent is conjugated to at least one ligand selected from the group consisting of cholesterol, biotin, vitamins, galactose derivatives or analogs, lactose derivatives or analogs, N-acetylgalactosamine derivatives or analogs, and N-acetylglucosamine (GalNAc) derivatives or analogs; preferably, the ligand is N-acetylglucosamine (GalNAc) derivatives or analogs.

**7.** The double-stranded RNAi agent according to claim 6, wherein the ligand is linked to the 3'-terminus, the 5'-terminus and/or internally within the strand of the double-stranded RNAi agent; preferably, the ligand is linked to the 3'-terminus of the sense strand of the double-stranded RNAi agent.

**8.** A double-stranded RNAi agent comprising:

(1) an antisense strand composed of a nucleotide sequence having at least 90%, preferably 95%, 96%, 97%, 98%, 99% or 100% identity to the nucleotide sequence UmsGfsUmAmCmUfCmUmCmAmUmUmGmUfGmGfAmUmGmAmCmsGmsAm (SEQ ID NO: 7) and a sense strand composed of a nucleotide sequence having at least 90%, preferably 95%, 96%, 97%, 98%, 99% or 100% identity to the nucleotide sequence GmsUmsCmAmUmCmCfAmCfAfAfUmGmAmGmAmGmUmAmCmAm (SEQ ID NO: 8);
(2) an antisense strand composed of a nucleotide sequence having at least 90%, preferably 95%, 96%, 97%, 98%, 99% or 100% identity to the nucleotide sequence GmsGfsUmAmCmUmCmUmCmAmUmUmGmUfGmGfAmUmGmAmCmsGmsAm (SEQ ID NO: 9) and a sense strand composed of a nucleotide sequence having at least 90%, preferably 95%, 96%, 97%, 98%, 99% or 100% identity to the nucleotide sequence GmsUmsCmAmUmCmCfAmCfAfAfUmGmAmGmAmGmUmAmCmCm (SEQ ID NO: 10);
(3) an antisense strand composed of a nucleotide sequence having at least 90%, preferably 95%, 96%, 97%, 98%, 99% or 100% identity to the nucleotide sequence GmsAfsAmCmCmUmGmUmCmAmAmUmCmUfUmCfUmCmAmGmCmsAmsGm (SEQ ID NO: 11) and a sense strand composed of a nucleotide sequence having at least 90%, preferably 95%, 96%, 97%, 98%, 99% or 100% identity to the nucleotide sequence GmsCmsUmGmAmGmAfAmGfAfUfUmGmAmCmAmGmGmUmUmCm (SEQ ID NO: 12);
(4) an antisense strand composed of a nucleotide sequence having at least 90%, preferably 95%, 96%, 97%, 98%, 99% or 100% identity to the nucleotide sequence UmsGfsAmAmCmCmUmGmUmCmAmAmUmCfUmUfCmUmCmAmGmsCmsAm (SEQ ID NO: 13) and a sense strand composed of a nucleotide sequence having at least 90%, preferably 95%, 96%, 97%, 98%, 99% or 100% identity to the nucleotide sequence CmsUmsGmAmGmAmAfGmAfUfUfGmAmCmAmGmGmUmUmCmAm (SEQ ID NO: 14);
(5) an antisense strand composed of a nucleotide sequence having at least 90%, preferably 95%, 96%, 97%, 98%, 99% or 100% identity to the nucleotide sequence UmsGfsAmUmCmAmUmAmCmAmCmAmGmCfAmAfAmCmAmGmGmsAmsAm (SEQ ID NO: 15) and a sense strand composed of a nucleotide sequence having at least 90%, preferably 95%, 96%, 97%, 98%, 99% or 100% identity to the nucleotide sequence CmsCmsUmGmUmUmUfGmCfUfGfUmGmUmAmUmGmAmUmCmAm (SEQ ID NO: 16);
(6) an antisense strand composed of a nucleotide sequence having at least 90%, preferably 95%, 96%, 97%, 98%, 99% or 100% identity to the nucleotide sequence UmsGfsAmCmAmCmAmUmCmGmCmUmGmAfUmUfUmGmUmCmCmsGmsGm (SEQ ID NO: 17) and a sense strand composed of a nucleotide sequence having at least 90%, preferably 95%, 96%, 97%, 98%, 99% or 100% identity to the nucleotide sequence GmsGmsAmCmAmAmAfUmCfAfGfCmGmAmUmGmUmGmUmCmAm (SEQ ID NO: 18);
(7) an antisense strand composed of a nucleotide sequence having at least 90%, preferably 95%, 96%, 97%, 98%, 99% or 100% identity to the nucleotide sequence AmsGfsAmAmGmAmAmAmAmGmGmUmGmGfGmAfGmAmCmUmGmsGmsGm (SEQ ID NO: 19) and a sense strand composed of a nucleotide sequence having at least 90%, preferably 95%, 96%, 97%, 98%, 99% or 100% identity to the nucleotide sequence CmsAmsGmUmCmUmCfCmCfAfCfCmUmUmUmUmCmUmUmCmUm (SEQ ID NO: 20);
(8) an antisense strand composed of a nucleotide sequence having at least 90%, preferably 95%, 96%, 97%, 98%, 99% or 100% identity to the nucleotide sequence CmsAfsUmUmAmGmAmAmGmAmAmAmAmGfGmUfGmGmGmAmGmsAmsCm (SEQ ID NO: 21) and a sense strand composed of a nucleotide sequence having at least 90%, preferably 95%, 96%, 97%, 98%, 99% or 100% identity to the nucleotide sequence CmsUmsCmCmCmAmCfCmUfUfUfUmCmUmUmCmUmAmAmUmGm (SEQ ID NO: 22);
(9) an antisense strand composed of a nucleotide sequence having at least 90%, preferably 95%, 96%, 97%,

98%, 99% or 100% identity to the nucleotide sequence UmsCfsAmAmAmGmUmCmGmAmCmUmCmAfUmUfAmGmAmAmGmsAmsAm (SEQ ID NO: 23) and a sense strand composed of a nucleotide sequence having at least 90%, preferably 95%, 96%, 97%, 98%, 99% or 100% identity to the nucleotide sequence CmsUmsUmCmUmAmAfUmGfAfGfUmCmGmAmCmUmUmUmGmAm (SEQ ID NO: 24);

(10) an antisense strand composed of a nucleotide sequence having at least 90%, preferably 95%, 96%, 97%, 98%, 99% or 100% identity to the nucleotide sequence AmsCfsAmCmUmUmAmGmAmCmCmAmAmGfGmAfGmAmAmAmCmsGmsGm (SEQ ID NO: 25) and a sense strand composed of a nucleotide sequence having at least 90%, preferably 95%, 96%, 97%, 98%, 99% or 100% identity to the nucleotide sequence GmsUmsUmUmCmUmCfCmUfUfGfGmUmCmUmAmAmGmUmGmUm (SEQ ID NO: 26); or

(11) an antisense strand composed of a nucleotide sequence having at least 90%, preferably 95%, 96%, 97%, 98%, 99% or 100% identity to the nucleotide sequence UmsUfsUmUmUmUmGmUmUmUmCmAmCmAfAmAfCmAmAmGmCmsUmsGm (SEQ ID NO: 27) and a sense strand composed of a nucleotide sequence having at least 90%, preferably 95%, 96%, 97%, 98%, 99% or 100% identity to the nucleotide sequence GmsCmsUmUmGmUmUfUmGfUfGfAmAmAmCmAmAmAmAmAmAm (SEQ ID NO: 28);

wherein Am, Um, Cm and Gm represent 2'-O-methyl-modified ribonucleotides A, U, C and G, respectively; Af, Uf, Cf and Gf represent 2'-fluoro-modified ribonucleotides A, U, C and G, respectively; and (s) represents that two adjacent nucleotides are linked via a phosphorothioate backbone.

**9.** The double-stranded RNAi agent according to claim 8, wherein the double-stranded RNAi agent is conjugated to at least one ligand.

**10.** The double-stranded RNAi agent according to claim 9, wherein the ligand is an N-acetylglucosamine (GalNAc) derivative or analog; preferably, the N-acetylglucosamine (GalNAc) derivative or analog is linked to the 3'-terminus of the sense strand of the double-stranded RNAi agent; more preferably, the GalNAc has a structure represented by formula II:

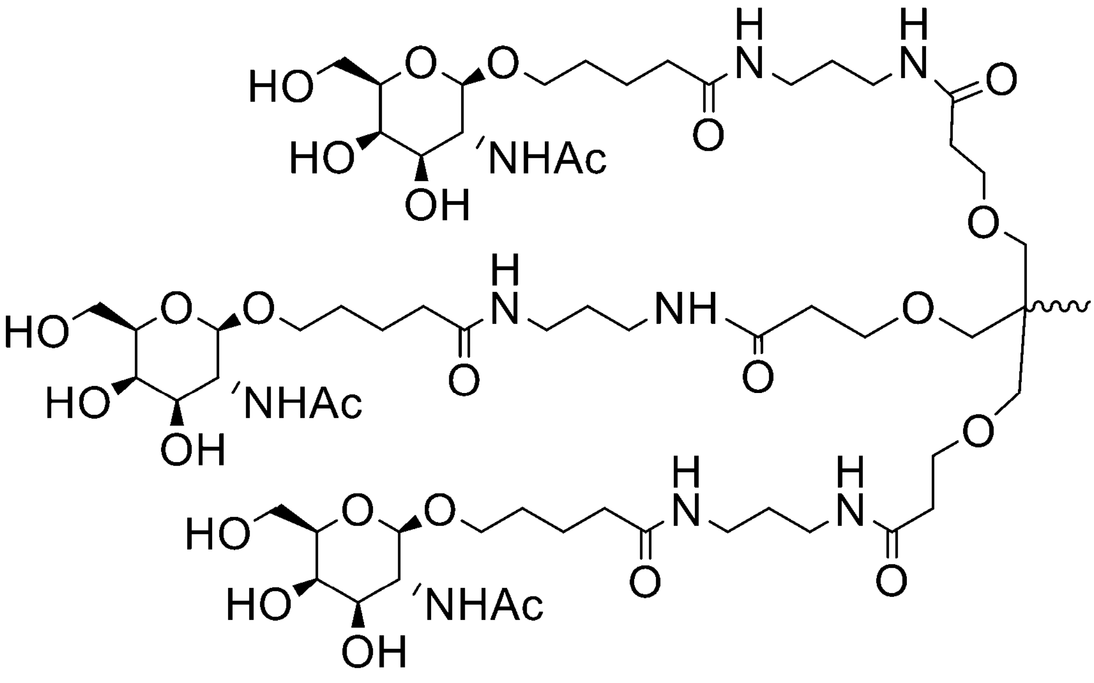

Formula II.

**11.** The double-stranded RNAi agent according to any one of claims 8 to 10, comprising:

(1) an antisense strand composed of a nucleotide sequence having at least 90%, preferably 95%, 96%, 97%, 98%, 99% or 100% identity to the nucleotide sequence UmsGfsUmAmCmUfCmUmCmAmUmUmGmUfGmGfAmUmGmAmCmsGmsAm (SEQ ID NO: 7) and a sense strand composed of a nucleotide sequence having at least 90%, preferably 95%, 96%, 97%, 98%, 99% or 100% identity to the nucleotide sequence GmsUmsCmAmUmCmCfAmCfAfAfUmGmAmGmAmGmUmAmCmAm-L96 (SEQ ID NO: 29);

(2) an antisense strand composed of a nucleotide sequence having at least 90%, preferably 95%, 96%, 97%, 98%, 99% or 100% identity to the nucleotide sequence GmsGfsUmAmCmUmCmUmCmAmUmUmGmUfGm GfAmUmGmAmCmsGmsAm (SEQ ID NO: 9) and a sense strand composed of a nucleotide sequence having at least 90%, preferably 95%, 96%, 97%, 98%, 99% or 100% identity to the nucleotide sequence GmsUmsCmAmUmCmCfAmCfAfAfUmGmAmGmAmGmUmAmCmCm-L96 (SEQ ID NO: 30);

(3) an antisense strand composed of a nucleotide sequence having at least 90%, preferably 95%, 96%, 97%, 98%, 99% or 100% identity to the nucleotide sequence GmsAfsAmCmCmUmGmUmCmAmAmUmCmUfUm CfUmCmAmGmCmsAmsGm (SEQ ID NO: 11) and a sense strand composed of a nucleotide sequence having at least 90%, preferably 95%, 96%, 97%, 98%, 99% or 100% identity to the nucleotide sequence GmsCmsUmGmAmGmAfAmGfAfUfUmGmAmCmAmGmGmUmUmCm-L96 (SEQ ID NO: 31);

(4) an antisense strand composed of a nucleotide sequence having at least 90%, preferably 95%, 96%, 97%, 98%, 99% or 100% identity to the nucleotide sequence UmsGfsAmAmCmCmUmGmUmCmAmAmUmCfUm UfCmUmCmAmGmsCmsAm (SEQ ID NO: 13) and a sense strand composed of a nucleotide sequence having at least 90%, preferably 95%, 96%, 97%, 98%, 99% or 100% identity to the nucleotide sequence CmsUmsGmAmGmAmAfGmAfUfUfGmAmCmAmGmGmUmUmCmAm-L96 (SEQ ID NO: 32);

(5) an antisense strand composed of a nucleotide sequence having at least 90%, preferably 95%, 96%, 97%, 98%, 99% or 100% identity to the nucleotide sequence UmsGfsAmUmCmAmUmAmCmAmCmAmGmCfAm AfAmCmAmGmGmsAmsAm (SEQ ID NO: 15) and a sense strand composed of a nucleotide sequence having at least 90%, preferably 95%, 96%, 97%, 98%, 99% or 100% identity to the nucleotide sequence CmsCmsUmGmUmUmUfGmCfUfGfUmGmUmAmUmGmAmUmCmAm-L96 (SEQ ID NO: 33);

(6) an antisense strand composed of a nucleotide sequence having at least 90%, preferably 95%, 96%, 97%, 98%, 99% or 100% identity to the nucleotide sequence UmsGfsAmCmAmCmAmUmCmGmCmUmGmAfUm UfUmGmUmCmCmsGmsGm (SEQ ID NO: 17) and a sense strand composed of a nucleotide sequence having at least 90%, preferably 95%, 96%, 97%, 98%, 99% or 100% identity to the nucleotide sequence GmsGmsAmCmAmAmAfUmCfAfGfCmGmAmUmGmUmGmUmCmAm-L96 (SEQ ID NO: 34);

(7) an antisense strand composed of a nucleotide sequence having at least 90%, preferably 95%, 96%, 97%, 98%, 99% or 100% identity to the nucleotide sequence AmsGfsAmAmGmAmAmAmAmGmGmUmGmGfGm AfGmAmCmUmGmsGmsGm (SEQ ID NO: 19) and a sense strand composed of a nucleotide sequence having at least 90%, preferably 95%, 96%, 97%, 98%, 99% or 100% identity to the nucleotide sequence CmsAmsGmUmCmUmCfCmCfAfCfCmUmUmUmUmCmUmUmCmUm-L96 (SEQ ID NO: 35);

(8) an antisense strand composed of a nucleotide sequence having at least 90%, preferably 95%, 96%, 97%, 98%, 99% or 100% identity to the nucleotide sequence CmsAfsUmUmAmGmAmAmGmAmAmAmAmGfGm UfGmGmGmAmGmsAmsCm (SEQ ID NO: 21) and a sense strand composed of a nucleotide sequence having at least 90%, preferably 95%, 96%, 97%, 98%, 99% or 100% identity to the nucleotide sequence CmsUmsCmCmCmAmCfCmUfUfUfUmCmUmUmCmUmAmAmUmGm-L96 (SEQ ID NO: 36);

(9) an antisense strand composed of a nucleotide sequence having at least 90%, preferably 95%, 96%, 97%, 98%, 99% or 100% identity to the nucleotide sequence UmsCfsAmAmAmGmUmCmGmAmCmUmCmAfUm UfAmGmAmAmGmsAmsAm (SEQ ID NO: 23) and a sense strand composed of a nucleotide sequence having at least 90%, preferably 95%, 96%, 97%, 98%, 99% or 100% identity to the nucleotide sequence CmsUmsUmCmUmAmAfUmGfAfGfUmCmGmAmCmUmUmUmGmAm-L96 (SEQ ID NO: 37);

(10) an antisense strand composed of a nucleotide sequence having at least 90%, preferably 95%, 96%, 97%, 98%, 99% or 100% identity to the nucleotide sequence AmsCfsAmCmUmUmAmGmAmCmCmAmAmGfGm AfGmAmAmAmCmsGmsGm (SEQ ID NO: 25) and a sense strand composed of a nucleotide sequence having at least 90%, preferably 95%, 96%, 97%, 98%, 99% or 100% identity to the nucleotide sequence GmsUmsUmUmCmUmCfCmUfUfGfGmUmCmUmAmAmGmUmGmUm-L96 (SEQ ID NO: 38); or

(11) an antisense strand composed of a nucleotide sequence having at least 90%, preferably 95%, 96%, 97%, 98%, 99% or 100% identity to the nucleotide sequence UmsUfsUmUmUmUmGmUmUmUmCmAmCmAfAm AfCmAmAmGmCmsUmsGm (SEQ ID NO: 27) and a sense strand composed of a nucleotide sequence having at least 90%, preferably 95%, 96%, 97%, 98%, 99% or 100% identity to the nucleotide sequence GmsCmsUmUmGmUmUfUmGfUfGfAmAmAmCmAmAmAmAmAmAm-L96 (SEQ ID NO: 39);

wherein Am, Um, Cm and Gm represent 2'-O-methyl-modified ribonucleotides A, U, C and G, respectively; Af, Uf, Cf and Gf represent 2'-fluoro-modified ribonucleotides A, U, C and G, respectively; and (s) represents that two adjacent nucleotides are linked via a phosphorothioate backbone, and L96 is linked to the 3'-terminus of the sense strand of the double-stranded RNAi agent and has a structural formula represented by formula III:

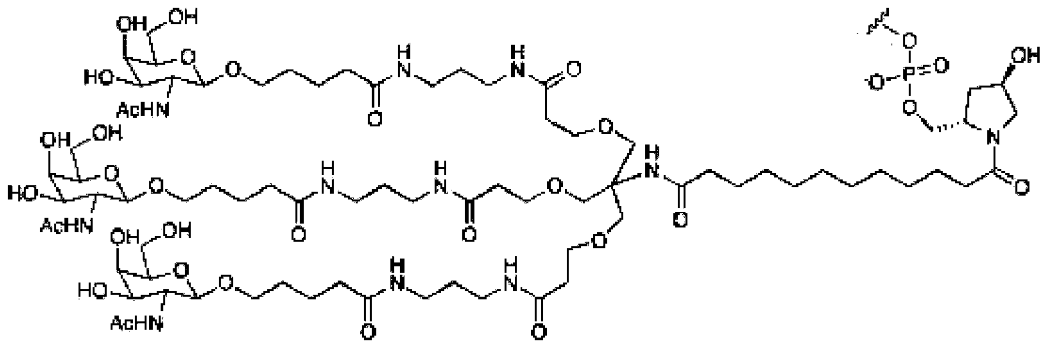

Formula III.

12. The double-stranded RNAi agent according to claim 11, wherein the double-stranded RNAi agent has a structure represented by formula IV:

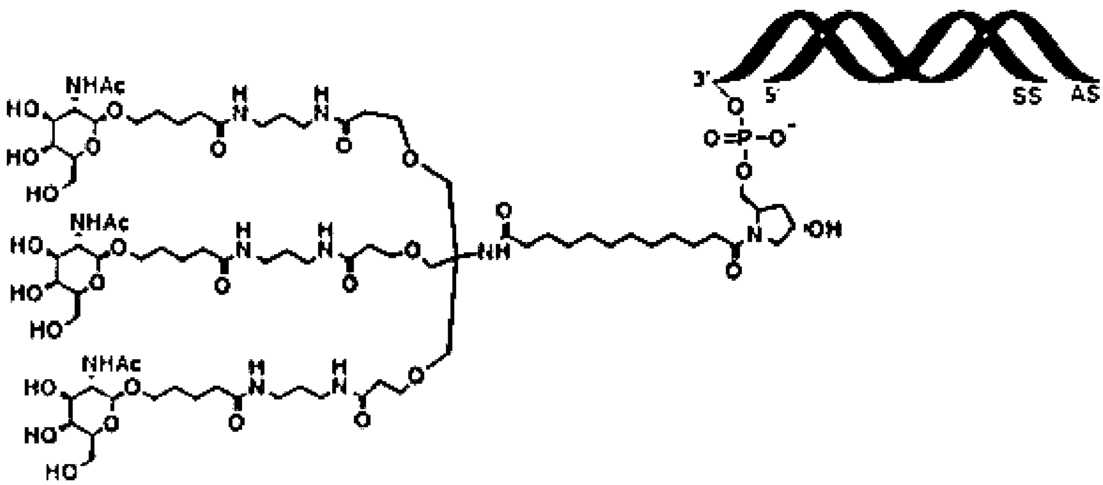

Formula IV.

13. A reagent or kit comprising the double-stranded RNAi agent according to any one of claims 1 to 12 or the DNA molecule according to claim 12.

14. A pharmaceutical composition comprising the double-stranded RNAi agent according to any one of claims 1 to 12.

15. Use of the double-stranded RNAi agent according to any one of claims 1 to 12 or the pharmaceutical composition according to claim 15 in preparation of a medicament for preventing and/or treating a disease mediated by *AGT* expression or alleviating symptoms of a disease mediated by an *AGT* gene.

16. The use according to claim 15, wherein the disease mediated by the AGT gene comprises hypertension, borderline hypertension, primary hypertension, secondary hypertension, hypertensive emergency, hypertensive urgency, isolated systolic or diastolic hypertension, pregnancy-associated hypertension, diabetic hypertension, resistant hypertension, refractory hypertension, paroxysmal hypertension, renovascular hypertension, Goldblatt hypertension, ocular hypertension, glaucoma, pulmonary hypertension, portal hypertension, systemic venous hypertension, systolic hypertension, labile hypertension; hypertensive heart disease, hypertensive nephropathy, atherosclerosis, arteriosclerosis, vasculopathy, diabetic nephropathy, diabetic retinopathy, chronic heart failure, cardiomyopathy, diabetic cardiac myopathy, glomerulosclerosis, coarctation of the aorta, aortic aneurism, ventricular fibrosis, Cushing's syndrome, and other glucocorticoid excess states including chronic steroid therapy, pheochromocytoma, reninoma, secondary aldosteronism and other mineralocorticoid excess states, sleep apnea, thyroid/parathyroid disease, heart failure, myocardial infarction, angina, stroke, diabetes mellitus, renal disease, renal failure, systemic sclerosis, intrauterine growth restriction (IUGR), and fetal growth restriction.

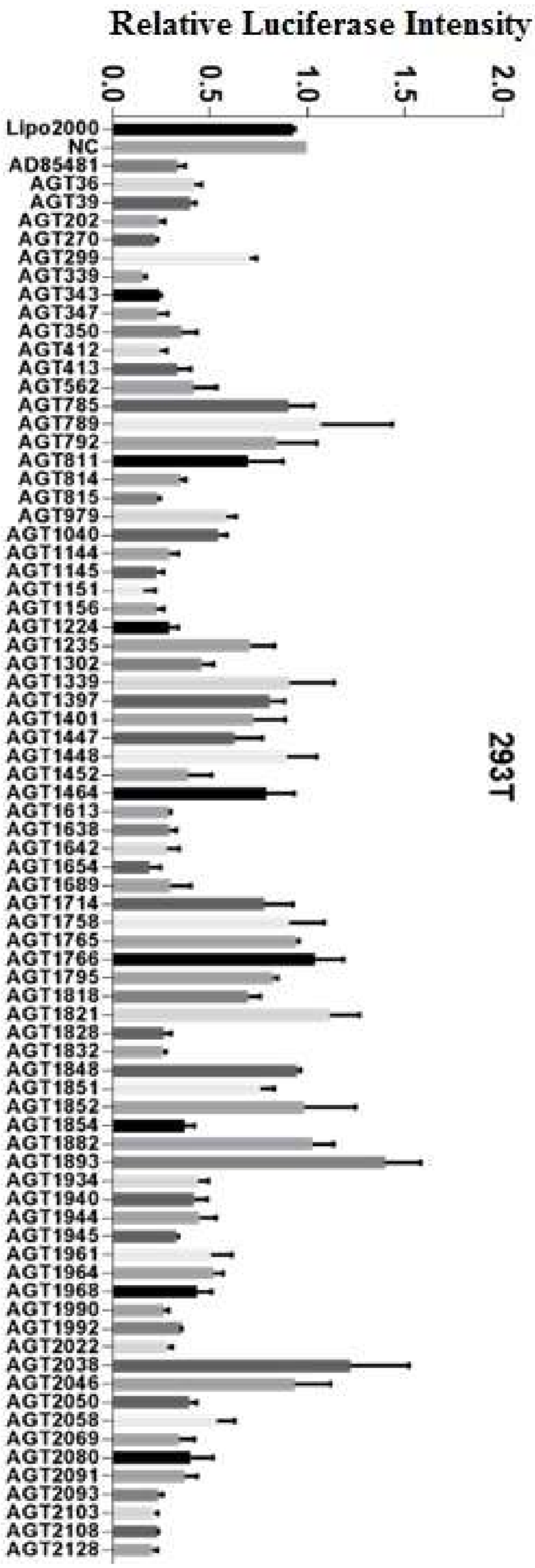
Relative Luciferase Intensity
0.0
0.5
1.0
1.5
2.0
293T
Lipo2000
NC
AD85481
AGT36
AGT39
AGT202
AGT270
AGT299
AGT339
AGT343
AGT347
AGT350
AGT412
AGT413
AGT562
AGT785
AGT789
AGT792
AGT811
AGT814
AGT815
AGT979
AGT1040
AGT1144
AGT1145
AGT1151
AGT1156
AGT1224
AGT1235
AGT1302
AGT1339
AGT1397
AGT1401
AGT1447
AGT1448
AGT1452
AGT1464
AGT1613
AGT1638
AGT1642
AGT1654
AGT1689
AGT1714
AGT1758
AGT1765
AGT1766
AGT1795
AGT1818
AGT1821
AGT1828
AGT1832
AGT1848
AGT1851
AGT1852
AGT1854
AGT1882
AGT1893
AGT1934
AGT1940
AGT1944
AGT1945
AGT1961
AGT1964
AGT1968
AGT1990
AGT1992
AGT2022
AGT2038
AGT2046
AGT2050
AGT2058
AGT2069
AGT2080
AGT2091
AGT2093
AGT2103
AGT2108
AGT2128

FIG. 1

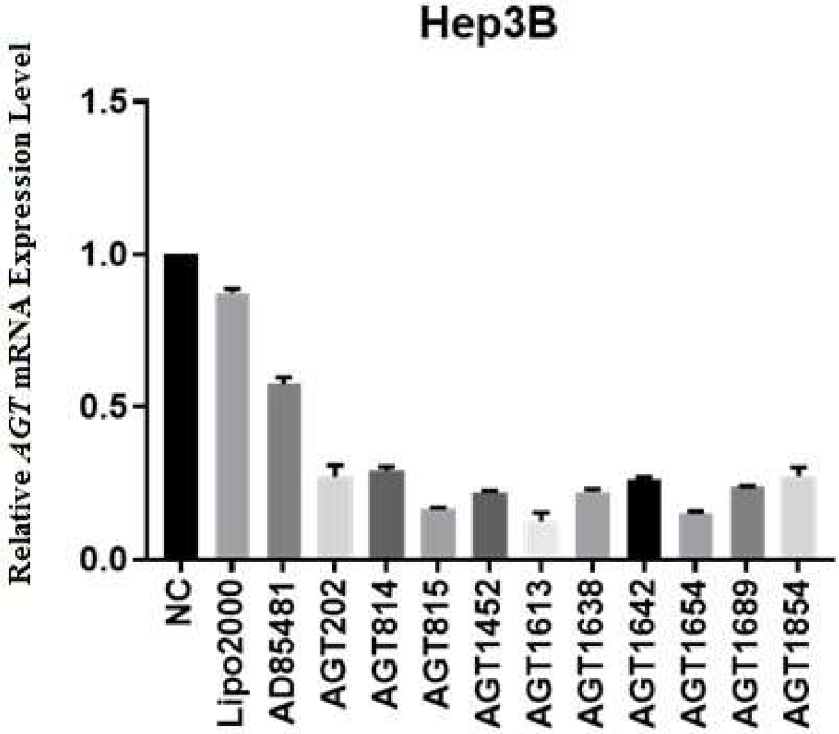
Hep3B
Relative AGT mRNA Expression Level
1.5
1.0
0.5
0.0
NC
Lipo2000
AD85481
AGT202
AGT814
AGT815
AGT1452
AGT1613
AGT1638
AGT1642
AGT1654
AGT1689
AGT1854

FIG. 2

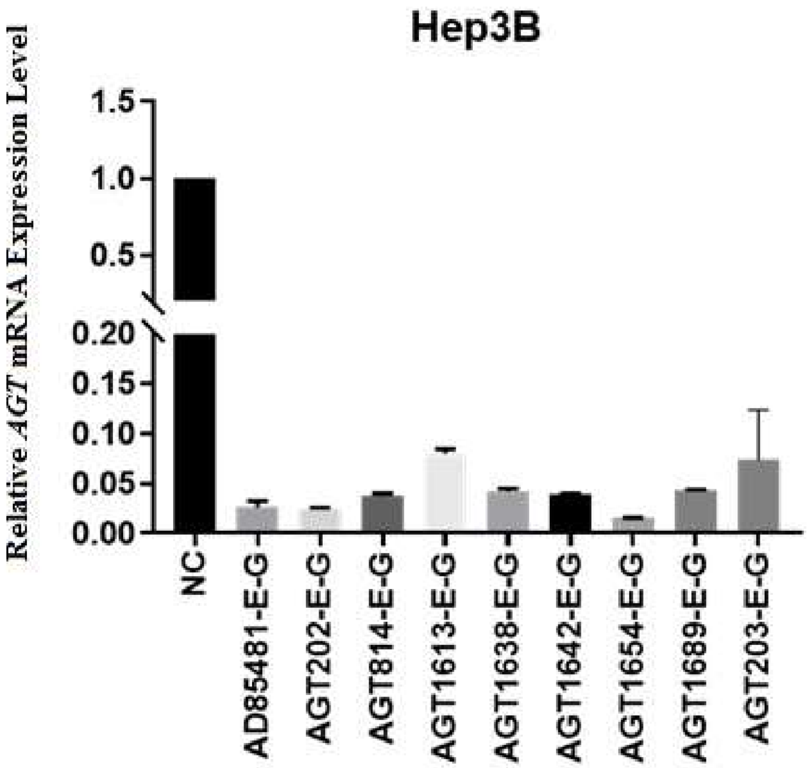
Hep3B
Relative AGT mRNA Expression Level
1.5
1.0
0.5
0.20
0.15
0.10
0.05
0.00
NC
AD85481-E-G
AGT202-E-G
AGT814-E-G
AGT1613-E-G
AGT1638-E-G
AGT1642-E-G
AGT1654-E-G
AGT1689-E-G
AGT203-E-G

FIG. 3

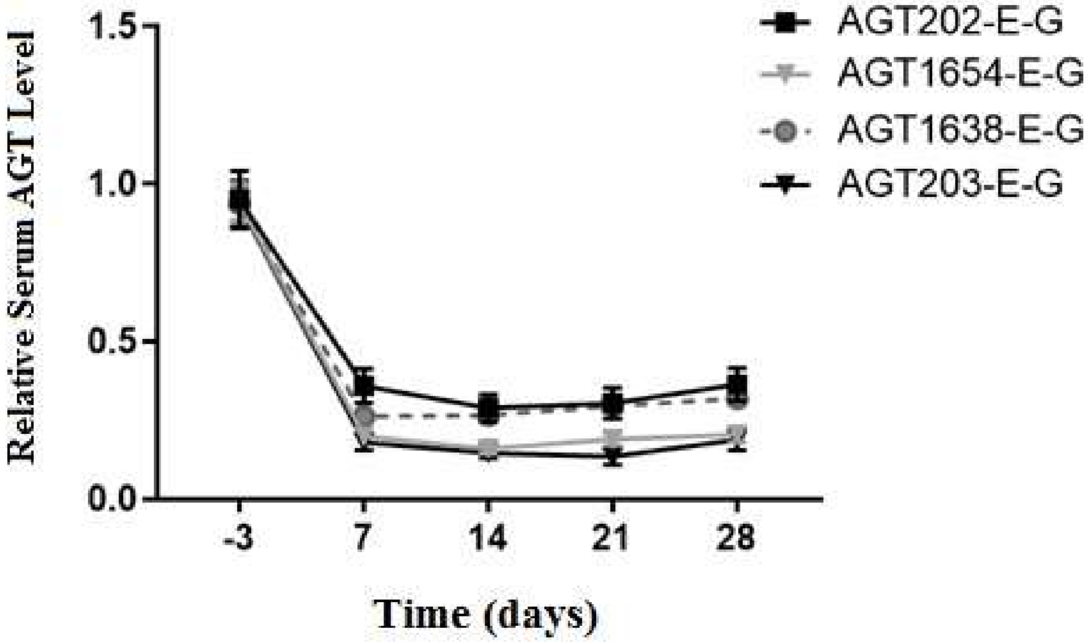
1.5
1.0
0.5
0.0
Relative Serum AGT Level
-3
7
14
21
28
Time (days)
AGT202-E-G
AGT1654-E-G
AGT1638-E-G
AGT203-E-G

FIG. 4

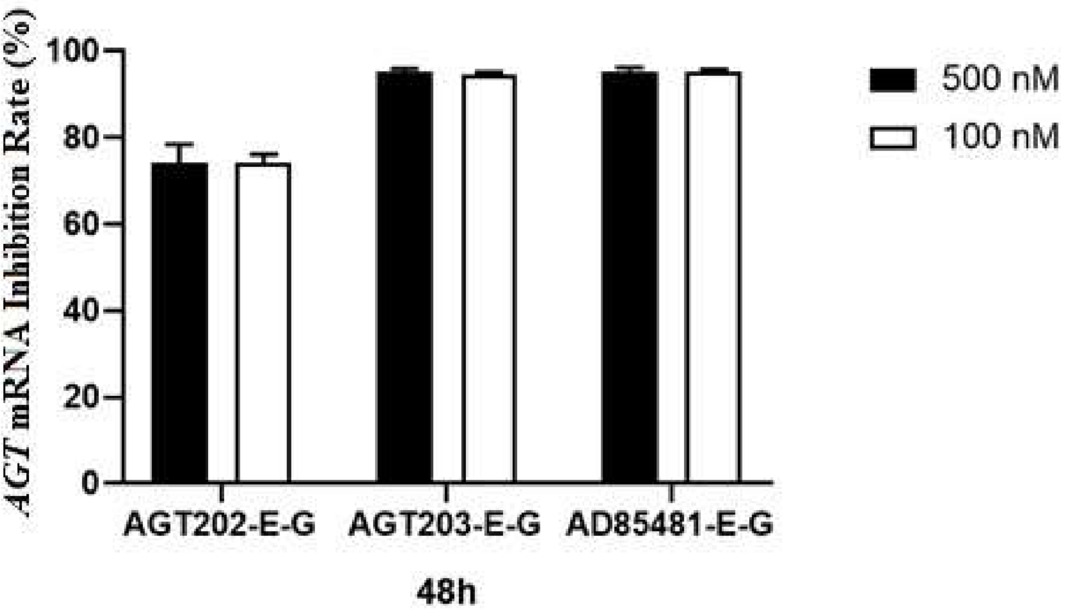
100
80
60
40
20
0
AGT mRNA Inhibition Rate (%)
AGT202-E-G
AGT203-E-G
AD85481-E-G
48h
500 nM
100 nM

FIG. 5

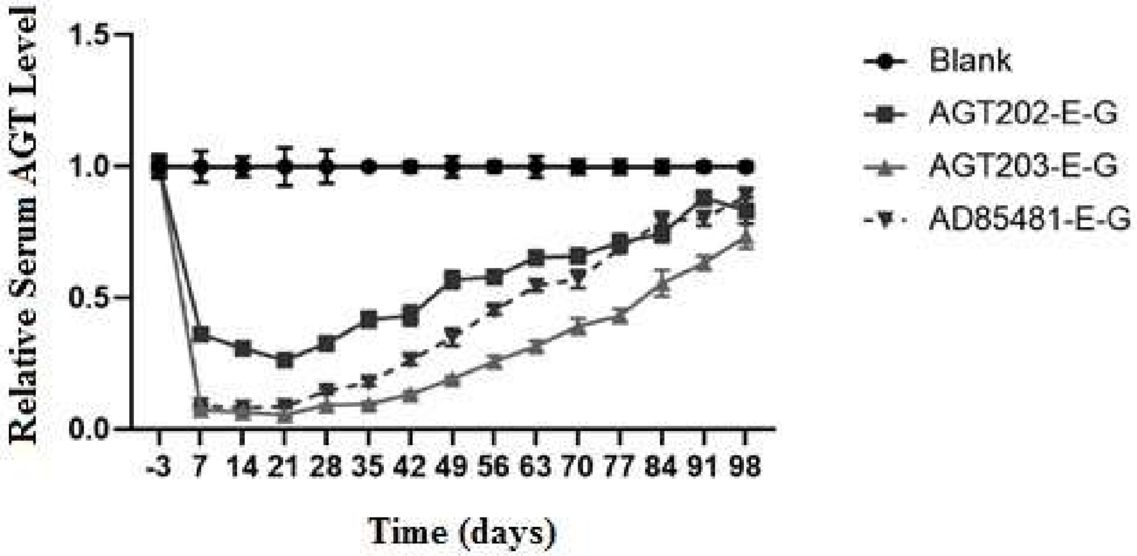
Relative Serum AGT Level
1.5
1.0
0.5
0.0
-3
7
14
21
28
35
42
49
56
63
70
77
84
91
98
Time (days)
Blank
AGT202-E-G
AGT203-E-G
AD85481-E-G

FIG. 6

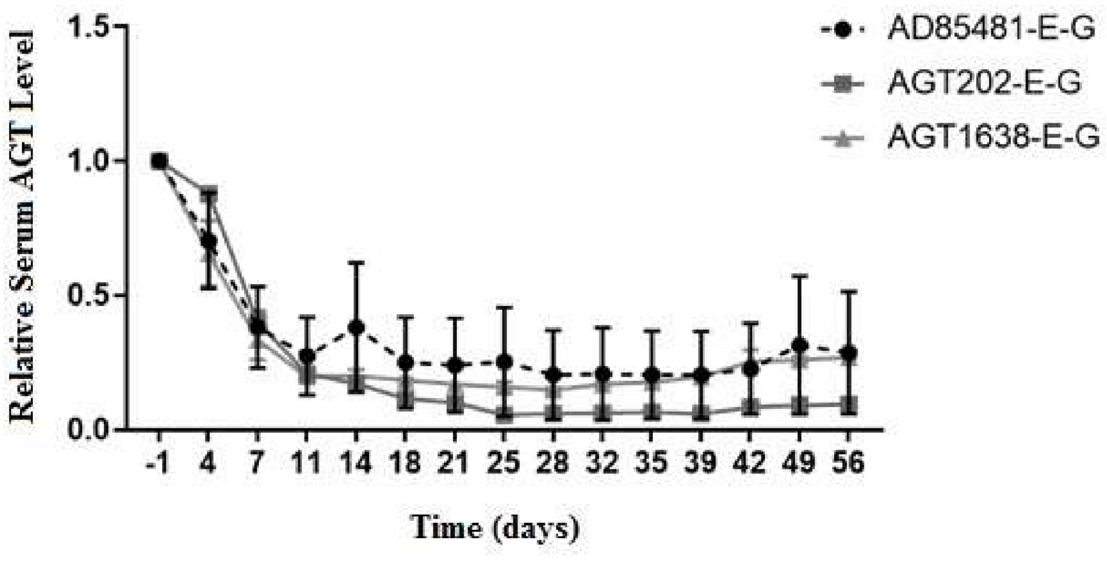
Relative Serum AGT Level
1.5
1.0
0.5
0.0
-1
4
7
11
14
18
21
25
28
32
35
39
42
49
56
Time (days)
AD85481-E-G
AGT202-E-G
AGT1638-E-G

FIG. 7

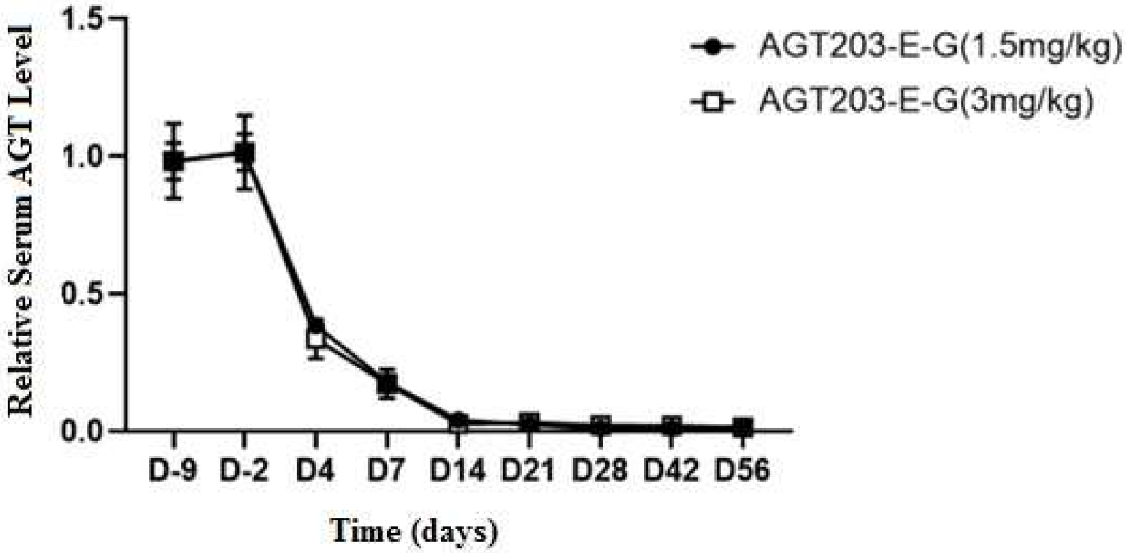
Relative Serum AGT Level
1.5
1.0
0.5
0.0
D-9 D-2 D4 D7 D14 D21 D28 D42 D56
Time (days)
AGT203-E-G(1.5mg/kg)
AGT203-E-G(3mg/kg)

FIG. 8

**INTERNATIONAL SEARCH REPORT**

International application No.
**PCT/CN2024/103896**

**A. CLASSIFICATION OF SUBJECT MATTER**

C12N 15/113(2010.01)i; A61K 31/713(2006.01)i; A61P 9/00(2006.01)i

According to International Patent Classification (IPC) or to both national classification and IPC

**B. FIELDS SEARCHED**

Minimum documentation searched (classification system followed by classification symbols)

IPC: C12N,A61K,A61P

Documentation searched other than minimum documentation to the extent that such documents are included in the fields searched

Electronic data base consulted during the international search (name of data base and, where practicable, search terms used)

CNTXT, DWPI, ENTXT, ENTXTC, VEN, WPABS, WPABSC, ISI Web of Knowledge, PubMed, CNKI, GenBank, 中国专利序列数据库, China Patents Sequence Database: 血管紧张素原, AGT, 抑制, 干扰, inhibit, RNAi, 双链RNAi, dsRNA, 有义链, sense strand, 反义链, antisense strand, 修饰, modification, 配体, ligand, 高血压, hypertension, SEQ ID NOs: 1-39

**C. DOCUMENTS CONSIDERED TO BE RELEVANT**

| Category* | Citation of document, with indication, where appropriate, of the relevant passages | Relevant to claim No. |
|---|---|---|
| X | CN 112313335 A (ALNYLAM PHARMACEUTICALS, INC.) 02 February 2021 (2021-02-02)<br>claims 1-78, and sequence listing | 1-16 |
| X | CN 112301031 A (ALNYLAM PHARMACEUTICALS, INC.) 02 February 2021 (2021-02-02)<br>claims 1-10, and sequence listing | 1-16 |
| A | CN 114981431 A (ALNYLAM PHARMACEUTICALS, INC.) 30 August 2022 (2022-08-30)<br>claims 1-97, and sequence listing | 1-16 |
| A | WO 2023014765 A1 (ALNYLAM PHARMACEUTICALS, INC.) 09 February 2023 (2023-02-09)<br>claims 1-81, and sequence listing | 1-16 |
| A | WO 2023278576 A1 (ALNYLAM PHARMACEUTICALS, INC.) 05 January 2023 (2023-01-05)<br>1-123 | 1-16 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 September 2024** | **23 September 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2024/103896

C. DOCUMENTS CONSIDERED TO BE RELEVANT

| Category* | Citation of document, with indication, where appropriate, of the relevant passages | Relevant to claim No. |
|---|---|---|
| A | UIJL, E. et al. "Strong and Sustained Antihypertensive Effect of Small Interfering RNA Targeting Liver Angiotensinogen" *Hypertension*, Vol. 73, 30 June 2019 (2019-06-30), pages 1249-1257 | 1-16 |

Form PCT/ISA/210 (second sheet) (July 2022)

**INTERNATIONAL SEARCH REPORT**

International application No.
**PCT/CN2024/103896**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/103896**

| Patent document cited in search report | Publication date (day/month/year) | Patent family member(s) | Publication date (day/month/year) |
|---|---|---|---|
| CN 112313335 A | 02 February 2021 | PE 20210114 A1 | 19 January 2021 |
| | | ES 2967713 T3 | 03 May 2024 |
| | | EP 3794122 A1 | 24 March 2021 |
| | | EP 3794122 B1 | 09 August 2023 |
| | | US 2021310006 A1 | 07 October 2021 |
| | | US 11834661 B2 | 05 December 2023 |
| | | CL 2023002870 A1 | 28 June 2024 |
| | | JP 2021522841 A | 02 September 2021 |
| | | JP 7346460 B2 | 19 September 2023 |
| | | MX 2020012048 A | 29 January 2021 |
| | | IL 278539 B | 01 June 2022 |
| | | ZA 202108348 B | 29 November 2023 |
| | | RS 64812 B1 | 29 December 2023 |
| | | IL 292877 A | 01 July 2022 |
| | | IL 292877 B1 | 01 April 2024 |
| | | IL 292877 B2 | 01 August 2024 |
| | | EP 4324520 A2 | 21 February 2024 |
| | | EP 4324520 A3 | 28 August 2024 |
| | | JP 2023182578 A | 26 December 2023 |
| | | CO 2020015314 A2 | 19 March 2021 |
| | | KR 20210010529 A | 27 January 2021 |
| | | WO 2019222166 A1 | 21 November 2019 |
| | | US 2021095290 A1 | 01 April 2021 |
| | | US 11015201 B2 | 25 May 2021 |
| | | SG 11202011144 QA | 30 December 2020 |
| | | CL 2021002326 A1 | 13 May 2022 |
| | | LT 3794122 T | 27 November 2023 |
| | | TW 202016304 A | 01 May 2020 |
| | | BR 112020022943 A2 | 17 February 2021 |
| | | PT 3794122 T | 22 November 2023 |
| | | CA 3100003 A1 | 21 November 2019 |
| | | FI 3794122 T3 | 07 November 2023 |
| | | HUE 064073 T2 | 28 February 2024 |
| | | SI 3794122 T1 | 29 February 2024 |
| | | US 2024191236 A1 | 13 June 2024 |
| | | CL 2020002865 A1 | 30 April 2021 |
| | | AU 2019269418 A1 | 07 January 2021 |
| | | PH 12020551904 A1 | 21 June 2021 |
| | | DK 3794122 T3 | 13 November 2023 |
| | | DK 3794122 T5 | 05 August 2024 |
| | | HRP 20231412 T1 | 16 February 2024 |
| | | PL 3794122 T3 | 11 March 2024 |
| CN 112301031 A | 02 February 2021 | SG 11201609376 SA | 29 December 2016 |
| | | WO 2015179724 A1 | 26 November 2015 |
| | | WO 2015179724 A8 | 18 February 2016 |
| | | WO 2015179724 A9 | 10 March 2016 |
| | | JP 2017517511 A | 29 June 2017 |
| | | JP 6811094 B2 | 13 January 2021 |
| | | BR 122020023687 B1 | 07 March 2023 |
| | | IL 281638 A | 31 May 2021 |

Form PCT/ISA/210 (patent family annex) (July 2022)

**INTERNATIONAL SEARCH REPORT**
**Information on patent family members**

International application No.
**PCT/CN2024/103896**

| Patent document cited in search report | Publication date (day/month/year) | Patent family member(s) | Publication date (day/month/year) |
|---|---|---|---|
| | | IL 281638 B1 | 01 March 2023 |
| | | IL 281638 B2 | 01 July 2023 |
| | | IL 248816 A0 | 31 January 2017 |
| | | IL 248816 B | 29 July 2021 |
| | | US 2023123192 A1 | 20 April 2023 |
| | | AU 2015264038 A1 | 01 December 2016 |
| | | AU 2015264038 A2 | 22 December 2016 |
| | | AU 2015264038 B2 | 11 February 2021 |
| | | EP 3739048 A1 | 18 November 2020 |
| | | US 2019298842 A1 | 03 October 2019 |
| | | US 10814007 B2 | 27 October 2020 |
| | | CA 2948381 A1 | 26 November 2015 |
| | | CA 2948381 C | 07 November 2023 |
| | | AU 2021202552 A1 | 27 May 2021 |
| | | BR 112016026950 A2 | 31 October 2017 |
| | | BR 112016026950 B1 | 07 March 2023 |
| | | KR 20170005130 A | 11 January 2017 |
| | | KR 102410687 B1 | 22 June 2022 |
| | | US 2017189541 A1 | 06 July 2017 |
| | | US 10238749 B2 | 26 March 2019 |
| | | JP 2022104985 A | 12 July 2022 |
| | | MX 2021006053 A | 06 July 2021 |
| | | KR 20220087576 A | 24 June 2022 |
| | | JP 2021063085 A | 22 April 2021 |
| | | JP 7101748 B2 | 15 July 2022 |
| | | SG 10202104570 TA | 29 June 2021 |
| | | EA 201692370 A1 | 31 March 2017 |
| | | EP 3146049 A1 | 29 March 2017 |
| | | EP 3146049 B1 | 26 February 2020 |
| | | US 2021046187 A1 | 18 February 2021 |
| | | US 11419942 B2 | 23 August 2022 |
| | | CA 3215908 A1 | 26 November 2015 |
| | | MX 2016015126 A | 23 February 2017 |
| | | ZA 201607666 B | 30 January 2019 |
| CN 114981431 A | 30 August 2022 | JP 2023502038 A | 20 January 2023 |
| | | CL 2022001256 A1 | 10 February 2023 |
| | | KR 20220115946 A | 19 August 2022 |
| | | MX 2022005692 A | 08 June 2022 |
| | | TW 202132568 A | 01 September 2021 |
| | | CO 2022006092 A2 | 20 May 2022 |
| | | BR 112022009216 A2 | 02 August 2022 |
| | | AU 2020382478 A1 | 02 June 2022 |
| | | US 2023002765 A1 | 05 January 2023 |
| | | PE 20230179 A1 | 01 February 2023 |
| | | IL 292865 A | 01 July 2022 |
| | | CA 3161703 A1 | 20 May 2021 |
| | | WO 2021096763 A1 | 20 May 2021 |
| | | WO 2021096763 A8 | 24 June 2021 |
| | | EP 4058577 A1 | 21 September 2022 |
| WO 2023014765 A1 | 09 February 2023 | MX 2024001445 A | 27 February 2024 |

Form PCT/ISA/210 (patent family annex) (July 2022)

**INTERNATIONAL SEARCH REPORT**
**Information on patent family members**

International application No.
**PCT/CN2024/103896**

| Patent document cited in search report | Publication date (day/month/year) | Patent family member(s) | Publication date (day/month/year) |
| --- | --- | --- | --- |
| | | TW 202337474 A | 01 October 2023 |
| | | CA 3228255 A1 | 09 February 2023 |
| | | EP 4381071 A1 | 12 June 2024 |
| | | IL 310295 A | 01 March 2024 |
| | | KR 20240042016 A | 01 April 2024 |
| | | AU 2022324003 A1 | 08 February 2024 |
| | | CO 2024001403 A2 | 26 February 2024 |
| | | PE 20241132 A1 | 24 May 2024 |
| WO 2023278576 A1 | 05 January 2023 | US 2024247266 A1 | 25 July 2024 |
| | | CA 3225469 A1 | 05 January 2023 |
| | | KR 20240026203 A | 27 February 2024 |
| | | MX 2023015489 A | 19 January 2024 |
| | | IL 309296 A | 01 February 2024 |
| | | AU 2022303164 A1 | 18 January 2024 |
| | | JP 2024527304 A | 24 July 2024 |
| | | EP 4363580 A1 | 08 May 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310826984
- CN 202410478014


### Non-patent literature cited in the description


- **ANDREW FIRE** ; **CRAIG MELLO et al.** *Caenorhabditis elegans (C. elegans)*, 1998